(19) Europäisches Patentamt

European Patent Office

Office européen des brevets

(11) **EP 4 109 466 A1**

(12) **EUROPÄISCHE PATENTANMELDUNG**

(43) Veröffentlichungstag:
    **28.12.2022 Patentblatt 2022/52**

(51) Internationale Patentklassifikation (IPC):
    **G16H 40/60** (2018.01)

(21) Anmeldenummer: **22164605.2**

(52) Gemeinsame Patentklassifikation (CPC):
    **G16H 40/60**

(22) Anmeldetag: **28.03.2022**

(84) Benannte Vertragsstaaten:
    **AL AT BE BG CH CY CZ DE DK EE ES FI FR GB GR HR HU IE IS IT LI LT LU LV MC MK MT NL NO PL PT RO RS SE SI SK SM TR**
    Benannte Erstreckungsstaaten:
    **BA ME**
    Benannte Validierungsstaaten:
    **KH MA MD TN**

(30) Priorität: **22.06.2021 DE 102021116062**

(71) Anmelder: **Hamilton Medical AG**
    **7402 Bonaduz (CH)**

(72) Erfinder:
    • **Reidt, Sascha**
      **7206 Zizers (CH)**
    • **Schranz, Christoph**
      **7304 Maienfeld (CH)**

(74) Vertreter: **Ruttensperger Lachnit Trossin Gomoll Patent- und Rechtsanwälte PartG mbB Arnulfstraße 58 80335 München (DE)**

(54) **BEATMUNGSVORRICHTUNG MIT AUTOMATISIERTER KORREKTUR PROXIMAL GEMESSENER ATEMGASFLUSSWERTE WÄHREND DES BEATMUNGSBETRIEBS**

(57)     Die vorliegende Erfindung betrifft eine Beatmungsvorrichtung (10) zur wenigstens unterstützenden künstlichen Beatmung eines Patienten (12), deren Datenverarbeitungsvorrichtung (19) dazu ausgebildet ist, auf Grundlage eines von einem distalen Atemgas-Flusssensor (48) ausgegeben Erfassungswerts, welcher einen inspiratorischen distalen Atemgasfluss in einer Kalibrations-Betriebssituation repräsentiert, nach Maßgabe eines ersten Modells einen inspiratorischen Kalibrations-Näherungswert zu ermitteln, welcher einen von einem proximalen Atemgas-Flusssensor (44) bei einer Erfassung des proximalen inspiratorischen Atemgasflusses in der Kalibrations-Betriebssituation erwarteten inspiratorischen Erfassungswert repräsentiert, wobei die Datenverarbeitungsvorrichtung (19) weiter dazu ausgebildet ist, unter Verwendung des Kalibrations-Nähe-rungswerts und eines in der Kalibrations-Betriebssituation vom proximalen Atemgas-Flusssensor (44) ausgegebenen realen inspiratorischen Erfassungswerts nach Maßgabe eines zweiten Modells den wenigstens einen inspiratorischen Fehlerparameter zu quantifizieren, und wobei die Datenverarbeitungsvorrichtung (19) noch weiter dazu ausgebildet ist, im weiteren Betrieb der Beatmungsvorrichtung (10) den wenigstens einen quantifizierten inspiratorischen Fehlerparameter auf vom proximalen Atemgas-Flusssensor (44) ausgegebene reale inspiratorische Erfassungswerte anzuwenden, um die tatsächlichen inspiratorischen Erfassungswerte des proximalen Atemgas-Flusssensors (44) in betragsmäßig weniger stark fehlerbehaftete korrigierte inspiratorische Erfassungswerte umzuwandeln.

Fig. 1

EP 4 109 466 A1

**Beschreibung**

Die vorliegende Erfindung betrifft eine Beatmungsvorrichtung zur wenigstens unterstützenden künstlichen Beatmung eines Patienten, umfassend:

- eine Atemgasleitung zur Leitung von inspiratorischem Atemgas zu einem Patienten hin und zur Leitung von exspiratorischem Atemgas vom Patienten weg,
- eine Atemgas-Fördervorrichtung zur Förderung von inspiratorischem Atemgas in der Atemgasleitung,
- einen näher am patientenseitigen Ende der Atemgasleitung angeordneten proximalen Atemgas-Flusssensor zur quantitativen Erfassung eines inspiratorischen und eines exspiratorischen proximalen Atemgasflusses in der Atemgasleitung und zur Ausgabe entsprechender Erfassungswerte,
- einen weiter vom patientenseitigen Ende der Atemgasleitung entfernt angeordneten distalen Atemgas-Flusssensor zur quantitativen Erfassung eines distalen Atemgasflusses in der Atemgasleitung,
- eine Datenverarbeitungsvorrichtung, welche zur Verarbeitung von an die Datenverarbeitungsvorrichtung ausgegebenen Erfassungswerten des proximalen und des distalen Atemgas-Flusssensors ausgebildet ist,
- eine mit der Datenverarbeitungsvorrichtung in Datenübertragungsverbindung stehende Datenspeicheranordnung, wobei in der Datenspeicheranordnung ein erstes Modell hinterlegt ist, welches als ein Betriebsmodell gestattet, für ein und dieselbe Betriebssituation der Beatmungsvorrichtung einen Atemgasfluss aus proximalem Atemgasfluss im Bereich des proximalen Atemgas-Flusssensors und distalem Atemgasfluss im Bereich des distalen Atemgas-Flusssensors auf Grundlage des jeweils anderen Atemgasflusses zu ermitteln,
- eine Steuervorrichtung zur Steuerung wenigstens der Atemgas-Fördervorrichtung und der Datenverarbeitungsvorrichtung,

wobei die Datenverarbeitungsvorrichtung dazu ausgebildet ist, Näherungswerte für Erfassungswerte des proximalen Atemgas-Flusssensors in einer Betriebssituation der Beatmungsvorrichtung nach Maßgabe des ersten Modells auf Grundlage von in der Betriebssituation erfassten Erfassungswerten des distalen Atemgas-Flusssensors näherungsweise zu ermitteln.

Eine solche Beatmungsvorrichtung ist aus der US 2011/0146681 A1 bekannt. Diese Druckschrift beschreibt das grundsätzlich bekannte Problem, dass ein proximaler Atemgas-Flusssensor, welcher in der Regel als Differenzdrucksensor ausgeführt ist, über seine Betriebsdauer im Beatmungsbetrieb hinweg seine Erfassungswerte-Ausgabecharakteristik verändert, gemäß welcher er einen Atemgasfluss erfasst und einen entsprechenden Erfassungswert ausgibt. Dies liegt daran, dass der proximale Atemgas-Flusssensor durch vom Atemgas mitgeführte Feuchtigkeit und durch Körperflüssigkeiten des beatmeten Patienten sowie durch vernebelte Medikamente, die bisweilen im Flusssensor auskristallisieren, in seinem Betrieb beeinflusst werden kann. Sich an betriebswesentlichen Bauteilen des Atemgas-Flusssensors niederschlagende Feuchtigkeit kann im Falle eines Differenzdrucksensors deren Beweglichkeit und somit die Erfassungswerte-Ausgabecharakteristik des Sensors verändern. Für Bauarten von Atemgas-Flusssensoren, bei denen es weniger auf Bauteilbeweglichkeit und mehr auf ein flussabhängiges thermisches Verhalten von Bauteilen ankommt, wie etwa bei Hitzdrahtanemometern, gilt im Wesentlichen das gleiche, da Feuchtigkeit im Flusssensor auch thermisches Verhalten von Bauteilen gegenüber einem trockenen Zustand verändern kann.

Die US 2011/0146681 A1 lehrt, den proximalen Atemgas-Flusssensor in Zeitabständen durch spontane Druckänderungen in der Atemgasleitung zu reinigen und am Atemgas-Flusssensor eine auf dem einschlägigen Fachgebiet als "Autozero" bezeichnete Null-Kalibration durchzuführen, um einen sich in der Regel ebenfalls durch Feuchtigkeitsbelastung einstellenden Driftfehler des proximalen Atemgas-Flusssensors zu beseitigen. Auch trockene Flusssensoren können einen Driftfehler zeigen.

Da der proximale Atemgas-Flusssensor während der Reinigung und während der Null-Kalibration keine oder keine brauchbaren Erfassungswerte an die Datenverarbeitungsvorrichtung überträgt, lehrt die US 2011/0146681 A1 zur Schließung dieser Erfassungslücke anstelle von Erfassungswerten des proximalen Atemgas-Flusssensors Erfassungswerte eines ebenfalls vorhandenen distalen Atemgas-Flusssensors zu verwenden und anhand des ersten Modells Erfassungswerte zu berechnen, welche in der betreffenden Betriebssituation von einem funktionierenden proximalen Atemgas-Flusssensor ausgegeben würden. Bei diesen anhand des ersten Modells auf Grundlage von Erfassungswerten des distalen Atemgas-Flusssensors, welcher gemäß der US 2011/0146681 A1 am patientenfernen Endbereich eines Exspirationsabschnitts der Atemgasleitung angeordnet ist, für den proximalen Atemgas-Flusssensor berechneten Erfassungswerten handelt es sich um Näherungswerte für die Erfassungswerte des proximalen Atemgas-Flusssensors, welche die in der Betriebssituation erwarteten tatsächlichen Erfassungswerte des proximalen Atemgas-Flusssensors mit für eine Beatmung von Patienten ausreichender Genauigkeit wiedergeben.

Nach der Phase der Reinigung und Null-Kalibration nimmt der proximale Atemgas-Flusssensor seinen Erfassungsbetrieb wieder auf und gibt Erfassungswerte für die von ihm erfassten proximalen Atemgasflüsse aus.

Aus der EP 3 384 948 A1 ist eine Beatmungsvorrichtung bekannt, welche während eines Beatmungsbetriebs eine nicht näher bezeichnete Kalibration eines proximalen Atemgas-Flusssensors durchführt. Hierzu benötigt die Beatmungsvorrichtung eine gesonderte Kalibrationsvorrichtung, welche bezüglich des inspiratorischen Atemgasflusses stromaufwärts des proximalen Atemgas-Flusssensors in die Atemgasleitung eingebaut ist, um während einer Kalibrationsphase des Atemgas-Flusssensors Strömungswege des Atemgases zu verändern. Die aus der EP 3 384 948 A1 bekannte Beatmungsvorrichtung weist keinen distalen Atemgas-Flusssensor auf, sondern kalibriert den proximalen Atemgas-Flusssensor offenbar anhand des Betriebs eines von der Atemgas-Fördervorrichtung der Beatmungsvorrichtung umfassten Gebläses.

Nachteilig an der aus der US 2011/0146681 A1 bekannten Lösung ist, dass zum einen das Reinigen des proximalen Atemgas-Flusssensors durch beispielsweise einen Druckstoß nicht unproblematisch ist, da dadurch im Atemgas-Flusssensor niedergeschlagene Flüssigkeiten, die überdies verkeimt sein können, unerwünschterweise dem Patienten angenähert werden könnten. Zumindest können diese den Flusssensor verunreinigenden Flüssigkeiten nicht ohne weiteres aus der Atemgasleitung entfernt werden. Sie verbleiben also in der Umgebung des proximalen Atemgas-Flusssensors und können diesen beeinträchtigen. Die Reinigungswirkung einer ausreichend milden reinigenden Druckschwankung auf den proximalen Atemgas-Flusssensor ist begrenzt und droht lediglich, im proximalen Atemgas-Flusssensor niedergeschlagene Flüssigkeit auf einen größeren räumlichen Bereich um den proximalen Atemgas-Flusssensor herum zu verteilen, von wo aus sie sich wieder zum Nachteil der Messgenauigkeit im proximalen Atemgas-Flussmessers sammeln kann.

Zum anderen wird punktuell durch die Null-Kalibration nur ein Driftfehler beseitigt, welcher die Erfassungswerte-Ausgabecharakteristik des proximalen Atemgas-Flusssensors nur verschiebt, jedoch nicht verändert. Die Null-Kalibration beseitigt für eine gewisse Zeit eine Ursache fehlerhafter Erfassungswerte, lässt jedoch die oben beschriebene feuchtigkeitsinduzierte Veränderung der Erfassungswerte-Ausgabecharakteristik des proximalen Atemgas-Flusssensors unberührt.

Die EP 3 384 948 A1 ist hier nur der Vollständigkeit halber erwähnt, da sie eine Kalibration eines proximal angeordneten Atemgas-Flusssensors während eines Beatmungsbetriebs offenbart. Allerdings ist der hierfür notwendige Aufwand durch den Einsatz der gesonderten Kalibrationsvorrichtung in der Atemgasleitung erheblich. Außerdem erfordert die Kalibration eines Atemgas-Flusssensors durch den Betrieb eines Gebläses ein ausreichend kalibriertes und von Einflüssen der Umgebung und des Betriebs ausreichend unverändertes Gebläse, sodass eine einmal für das Gebläse erstellte Kalibration auch nach längerem Betrieb der Beatmungsvorrichtung noch als gültig angenommen werden kann. Zeigen jedoch Bauteile des Gebläses Spuren von Verschleiß, gilt eine für das fabrikneue Gebläse erstellte Kalibration nicht mehr und der so entstandene, zunächst unbeachtliche Kalibrationsfehler des Gebläses wird durch die Kalibration des proximal angeordneten Atemgas-Flusssensors auf diesen übertragen. Somit wird der Kalibrationsfehler des Gebläses erheblich, da die Steuerung des Beatmungsbetriebs der bekannten Beatmungsvorrichtung maßgeblich auf Erfassungswerten des Atemgas-Flusssensors beruht.

Es ist außerdem, insbesondere an Beatmungsgeräten der Anmelderin, bekannt, Erfassungswerte eines distalen inspiratorischen Atemgas-Flusssensors heranzuziehen, um zu beurteilen, ob der proximale inspiratorische Atemgas-Flusssensor vorbestimmte Fehlergrenzen überschreitet oder nicht. Da jedoch das inspiratorische Atemgas nach der distalen Erfassung seines Flusses häufig konditioniert wird, also seine Feuchtigkeit und seine Temperatur geändert werden, und da außerdem ein gewisser, in der Fachwelt als "baseflow" bezeichneter Basisfluss unter Umgehung des Patienten vom inspiratorischen Teil der Atemgasleitung direkt in deren exspiratorischen Teil und von dort in eine Atemgassenke, in der Regel die Umgebungsatmosphäre des Beatmungsgeräts, strömt, gibt der vom distalen Atemgas-Flusssensor erfasste distale inspiratorische Atemgasfluss den einem Patienten verabreichten Atemgasfluss nur ungenau wieder. Daher ist eine zuverlässige und genaue Erfassung der tatsächlich beim Patienten ankommenden inspiratorischen Atemgasflüsse durch den distalen Sensor nicht möglich. Deshalb wird für eine Überwachung des künstlichen Beatmungsvorgangs der proximale Sensor verwendet.

Es ist Aufgabe der vorliegenden Erfindung, eine Beatmungsvorrichtung, wie sie eingangs dieser Anmeldung beschrieben ist, derart weiterzubilden, dass sie mit möglichst einfachen korrektiven Mitteln und mit möglichst geringer Auswirkung derselben auf einen Beatmungsbetrieb eines Patienten über eine möglichst lange Dauer den Beatmungsbetrieb mit möglichst hoher Genauigkeit aufrechterhalten kann.

Diese Aufgabe wird gemäß der vorliegenden Erfindung gelöst durch eine Beatmungsvorrichtung der eingangs genannten Art, bei welcher zusätzlich der distale Atemgas-Flusssensor zur quantitativen Erfassung des distalen inspiratorischen Atemgasflusses ausgebildet ist und bei welcher in der Datenspeicheranordnung ein zweites Modell hinterlegt ist, welches als ein inspiratorisches Fehlermodell gestattet, für ein und dieselbe Betriebssituation einen Erfassungswert aus einem theoretischen inspiratorischen Erfassungswert, wie er von einem hypothetischen, fehlerfrei arbeitenden proximalen Atemgas-Flusssensor als Ergebnis einer Erfassung des inspiratorischen proximalen Atemgasflusses an die Datenverarbeitungsvorrichtung ausgegeben würde, und einem realen inspiratorischen Erfassungswert, wie er vom proximalen Atemgas-Flusssensor als Ergebnis einer Erfassung des inspiratorischen proximalen Atemgasflusses tatsächlich an die Datenverarbeitungsvorrichtung ausgegeben wird, unter Verwendung wenigstens eines inspiratorischen Fehlerparameters auf Grundlage des jeweils anderen Erfassungswerts zu ermitteln,

wobei die Datenverarbeitungsvorrichtung dazu ausgebildet ist, auf Grundlage eines vom distalen Atemgas-Flusssensor an die Datenverarbeitungsvorrichtung ausgegeben Erfassungswerts, welcher einen distalen inspiratorischen Atemgasfluss in einer Kalibrations-Betriebssituation repräsentiert, nach Maßgabe des ersten Modells einen inspiratorischen Kalibrations-Näherungswert zu ermitteln, welcher einen vom proximalen Atemgas-Flusssensor bei einer Erfassung des proximalen inspiratorischen Atemgasflusses in der Kalibrations-Betriebssituation erwarteten inspiratorischen Erfassungswert repräsentiert,

wobei die Datenverarbeitungsvorrichtung weiter dazu ausgebildet ist, unter Verwendung des Kalibrations-Näherungswerts als dem theoretischen inspiratorischen Erfassungswert und eines in der Kalibrations-Betriebssituation vom proximalen Atemgas-Flusssensor an die Datenverarbeitungsvorrichtung ausgegebenen realen inspiratorischen Erfassungswerts nach Maßgabe des zweiten Modells den wenigstens einen inspiratorischen Fehlerparameter zu quantifizieren, und

wobei die Datenverarbeitungsvorrichtung noch weiter dazu ausgebildet ist, im weiteren Betrieb der Beatmungsvorrichtung den wenigstens einen quantifizierten inspiratorischen Fehlerparameter auf vom proximalen Atemgas-Flusssensor an die Datenverarbeitungsvorrichtung ausgegebene reale inspiratorische Erfassungswerte anzuwenden, um die tatsächlichen inspiratorischen Erfassungswerte des proximalen Atemgas-Flusssensors in betragsmäßig weniger stark fehlerbehaftete korrigierte inspiratorische Erfassungswerte umzuwandeln.

Die vorliegende Erfindung wird nachfolgend anhand einer Ausbildung einzelner Vorrichtungen, wie Flusssensoren, der Datenverarbeitungsvorrichtung und der Steuervorrichtung, zur Durchführung bestimmter Abläufe erläutert. Die dabei beschriebenen Abläufe sind auch als Beschreibung eines die oben genannte Aufgabe lösendes Verfahren, insbesondere Betriebsverfahren einer Beatmungsvorrichtung, zu verstehen. Die zur Ausbildung der einzelnen Abläufe ausgebildeten Vorrichtungen sind dabei die diese Abläufe während der Ausführung des Verfahrens durchführenden Vorrichtungen.

Durch die Verwendung eines distalen Atemgas-Flusssensors welcher zur Erfassung des inspiratorischen Atemgasflusses ausgebildet ist, kann ein vom proximalen Atemgas-Flusssensor ausgegebener inspiratorischer Erfassungswert eines von der Atemgas-Fördervorrichtung definiert erzeugten inspiratorischen Atemgasflusses mit einem in der gleichen Betriebssituation vom distalen Atemgas-Flusssensor an anderer Stelle der Atemgasleitung erfassten und ausgegebenen inspiratorischen Erfassungswert verglichen werden. Der distale Atemgas-Flusssensor kann auch zur Erfassung des inspiratorischen Atemgasflusses ausgebildet sein, ist vorzugsweise jedoch nur zur Erfassung des inspiratorischen Atemgasflusses ausgebildet und angeordnet.

Im Gegensatz zu einem Vergleich von Erfassungswerten des distalen und des proximalen Atemgas-Flusssensors im Exspirationspfad der Atemgasleitung können durch eine Erfassung des inspiratorischen Atemgasflusses durch sowohl den distalen wie auch den proximalen Atemgas-Flusssensor an unterschiedlichen Orten erfasste Erfassungswerte desselben durch die Atemgas-Fördervorrichtung bestimmten Atemgasflusses bevorzugt quantitativ, wenigstens jedoch qualitativ verglichen werden. Dabei kann selbst eine verschleißbehaftete Atemgas-Fördervorrichtung während der Erfassungen zur Quantifizierung des inspiratorischen Fehlerparameters immer noch einen betragsmäßig konstanten inspiratorischen Atemgasfluss erzeugen. Die Ausbildung der Atemgas-Fördervorrichtung zur Erzeugung eines betragsmäßig konstanten inspiratorischen Atemgasflusses und die Ansteuerung durch die Steuervorrichtung hierzu in der Kalibrations-Betriebssituation ist eine bevorzugte Weiterbildung der vorliegenden Erfindung. Der exspiratorische Atemgasfluss ist im Wesentlichen durch die Physis des beatmeten Patienten determiniert und unterliegt während einer Exspirationsphase eines Atemhubs starken betragsmäßigen Veränderungen. Derartige Veränderungen des Atemgasflusses über die Zeit können sich in unterschiedlicher elastischer Dehnung der Atemgasleitung und damit in zeitlich unterschiedlich starker Speicherung von Atemgas des Atemgasflusses in der Atemgasleitung niederschlagen. Bevorzugt ist daher die Atemgas-Fördervorrichtung zur Erzeugung eines betragsmäßig konstanten inspiratorischen Atemgasstroms während dessen Erfassung zur Quantifizierung des inspiratorischen Fehlerparameters ausgebildet. Dies verbessert auch die Aussagekraft des ersten Modells, sodass der durch das erste Modell auf Grundlage des distalen inspiratorischen Erfassungswerts ermittelte Näherungswert einen geringeren betragsmäßigen Abstand vom wahren proximalen inspiratorischen Erfassungswert des proximalen Atemgas-Flusssensors hat als bei einem stark transienten Atemgasfluss. Das erste Modell kann für eine gegebene Beatmungsvorrichtung unter Laborbedingungen ermittelt und verifiziert werden, sodass die von ihm auf Grundlage eines distalen inspiratorischen Erfassungswerts vorhergesagten Näherungswerte sich von dem tatsächlichen proximalen inspiratorischen Erfassungswert eines korrekt arbeitenden proximalen Arbeitsgas-Flusssensors betragsmäßig um einen vorbestimmten Prozentsatz, etwa um nicht mehr als 15 %, vorzugsweise um nicht mehr als 10 % und besonders bevorzugt um nicht mehr als 5 % bezogen auf den tatsächlichen Erfassungswert des korrekt arbeitenden proximalen Arbeitsgas-Flusssensors unterscheiden.

Die Referenz eines fehlerfreien, korrekt arbeitenden proximalen Arbeitsgas-Flusssensors im Rahmen der vorliegenden Beschreibung ist ein ohne Fabrikationsfehler gefertigter fabrikneuer proximaler Arbeitsgas-Flusssensor, vorzugsweise ein Differenzdrucksensor. Ein solcher proximaler Atemgas-Flusssensor ist in der vorliegenden Anmeldung auch als hypothetischer fehlerfrei arbeitender proximaler Atemgas-Flusssensor bezeichnet.

Im Beatmungsbetrieb treten am proximalen Atemgas-Flusssensor stets Fehlereinflussquellen auf, die aus ein und derselben Gruppe möglicher Fehlereinflussquellen stammen. Diese umfassen sich an Bauteilen des proximalen Atemgas-Flusssensors niederschlagende Feuchtigkeit, wie etwa Sputum, kondensierende Feuchtigkeit einschließlich aerosoler Medikamente, und sich im proximalen Atemgas-Flusssensor ansammelnde Feuchtigkeit im Wesentlichen gleichen Ursprungs oder/und auskristallisierende, ursprünglich ins Atemgas vernebelte Medikamente. Die Feuchtigkeit kann dem inspiratorischen oder/und dem exspiratorischen Atemgas durch Kondensation entzogen worden sein oder/und sie kann durch vom Patienten in die Atemgasleitung abgegebene Körperflüssigkeiten gebildet sein. Bei dem bevorzugten Ausführungsbeispiel des proximalen Atemgas-Flusssensors, einem Differenzdruck-Flusssensor, bewirkt übermäßige Feuchtigkeit eine Änderung der Beweglichkeit eines Strömungswiderstandsbauteils im Flusssensor und allgemein des Strömungsverhaltens von Atemgas im Flusssensor. Abhängig vom Atemgasfluss durch den Differenzdruck-Flusssensor wird das Strömungswiderstandsbauteil unterschiedlich stark ausgelenkt, wodurch sich ein vom Atemgasfluss abhängiger, betragsmäßig unterschiedlicher Strömungswiderstand im Flusssensor ausbildet. Wiederum abhängig vom jeweiligen Strömungswiderstand im Flusssensor ändert sich der Druckabfall über das Strömungswiderstandsbauteil hinweg und damit die Druckdifferenz zwischen einem Erfassungsort stromaufwärts und einem Erfassungsort stromabwärts des Strömungswiderstandsbauteils. Abhängig von der erfassten Druckdifferenz bestimmt die Datenverarbeitungsvorrichtung betragsmäßig den die Druckdifferenz bewirkenden Atemgasfluss. Ändert sich folglich das Verhalten des Strömungswiderstandsbauteils, ändert sich auch die Erfassungswerte-Ausgabecharakteristik des proximalen Atemgas-Flusssensors.

Da die Fehlereinflussquellen stets auf im Wesentlichen derselben Schar von Ursachen beruhen, können diese mit ausreichender Genauigkeit und Wiederholbarkeit durch das zweite Modell berücksichtigt werden. Die bekannten Fehlereinflussquellen bestimmen im Wesentlichen die Struktur des zweiten Modells. Die Struktur des zweiten Modells ist somit ebenso bekannt und kann im Vorfeld des Einsatzes der Beatmungsvorrichtung im Labor an baugleichen Beatmungsvorrichtungen ermittelt und verifiziert werden. Das Ausmaß, mit welcher die Fehlereinflussquellen auftreten, bestimmt die quantitative Parametrisierung des zweiten Modells. Hierzu werden weiter unten detaillieren der Ausführungen gemacht.

Das zweite Modell gestattet grundsätzlich die Ermittlung eines theoretischen inspiratorischen Erfassungswerts, wie er von dem oben genannten hypothetischen, fehlerfrei arbeitenden proximalen Atemgas-Flusssensor als Ergebnis einer Erfassung des inspiratorischen proximalen Atemgasflusses an die Datenverarbeitungsvorrichtung ausgegeben würde. Eine Ausgabe von Druckwerten stromaufwärts und stromabwärts des Strömungswiderstandsbauteils des als proximaler Atemgas-Flusssensor bevorzugten Differenzdruck-Flusssensors und deren Verarbeitung zu einem Atemgas-Flusswert durch die Datenverarbeitungsvorrichtung ist eine Ausgabe von Erfassungswerten des proximalen Atemgas-Flusssensors an die Datenverarbeitungsvorrichtung.

Die Datenverarbeitungsvorrichtung ist nun in der Lage und dazu ausgebildet, auf Grundlage eines Ergebnisses einer Erfassung des inspiratorischen Atemgasflusses durch den distalen Atemgas-Flusssensor unter Verwendung des ersten Modells zu ermitteln, welches theoretische Erfassungsergebnis ein hypothetischer fehlerfrei arbeitender proximaler Atemgas-Flusssensor liefern würde.

Da gewünscht ist, dass der proximale Atemgas-Flusssensor tatsächlich innerhalb eines vorbestimmten Toleranzbereichs das theoretische Erfassungsergebnis auch als reales Erfassungsergebnis liefert, kann unter Verwendung des zweiten Modells auf Grundlage des bekannten realen Erfassungsergebnisses des tatsächlich arbeitenden proximalen Atemgas-Flusssensors sowie auf Grundlage des unter Verwendung des ersten Modells ermittelten theoretischen Erfassungsergebnisses der wenigstens eine inspiratorische Fehlerparameter quantifiziert werden, sodass das erste Modell mit dem wenigstens einen quantifizierten inspiratorischen Fehlerparameter den tatsächlich erhaltenen realen inspiratorischen Erfassungswert auf den theoretischen inspiratorischen Fehlerparameter abbildet.

Die Benennung der Betriebssituation, in welcher diese Quantifizierung des wenigstens einen inspiratorischen Fehlerparameters stattfindet, als "Kalibrations-Betriebssituation" soll lediglich zum Ausdruck bringen, dass im Betrieb der Beatmungsvorrichtung vorübergehend die Datenverarbeitung zur Quantifizierung des wenigstens einen inspiratorischen Fehlerparameters abläuft. Während dieser Quantifizierung wird der Patient weiter beatmet. Es findet für die Quantifizierung des wenigstens einen inspiratorischen Fehlerparameters keine Abzweigung eines Teils des inspiratorischen und auch des exspiratorischen Atemgasflusses statt. Ebenso wenig wird die Beatmung des Patienten unterbrochen oder werden Atemgasflüsse durch eine irgendwie geartete Kalibrationsvorrichtung vorübergehend umgeleitet. Für die vorliegend beschriebene vorteilhafte Quantifizierung des wenigstens einen Fehlerparameters reicht eine gewöhnliche Beatmungsvorrichtung, wie sie eingangs beschrieben ist, aus.

Ist der wenigstens eine inspiratorische Fehlerparameter quantifiziert, kann ab diesem Zeitpunkt unter Verwendung des zweiten Modells und auf Grundlage der realen inspiratorischen Erfassungswerte des proximalen Atemgas-Flusssensors jedem realen inspiratorischen Erfassungswert ein korrigierter inspiratorischer Erfassungswert zugeordnet werden, welcher näher bei dem theoretischen inspiratorischen Erfassungswert liegt, den ein von Fehlerquellen unbelasteter, beispielsweise gemäß vorgegebener Qualitätssicherungsstandards fehlerfrei hergestellter fabrikneuer, proximaler Atemgas-Flusssensor unter gleichen Betriebsbedingungen liefern würde. Unter Verwendung dieser korrigierten inspiratorischen Erfassungswerte kann der weitere Beatmungsbetrieb der Beatmungsvorrichtung selbst bei Verwendung eines fehlerbelasteten proximalen Atemgas-Flusssensors mit gegenüber dem Stand der Technik erhöhter Genauigkeit fortgesetzt werden.

Die Quantifizierung des wenigstens einen inspiratorischen Fehlerparameters und dessen anschließende Verwendung zur Korrektur der inspiratorischen Erfassungswerte des proximalen Atemgas-Flusssensors ist nachfolgend auch als "inspiratorische Kalibration" bezeichnet.

Die Datenverarbeitungsvorrichtung oder/und die Steuervorrichtung kann eine solche Quantifizierung des wenigstens einen inspiratorischen Fehlerparameters nach vorgegebenen Intervallen, etwa nach einem vorbestimmten Zeitintervall oder nach einer vorgegebenen Anzahl von Atemhüben, automatisiert durchführen und so für eine automatisierte Korrektur der proximalen inspiratorischen Erfassungswerte sorgen.

Wie bereits dargelegt wurde, kann die oben beschriebene inspiratorische Kalibration des proximalen Atemgas-Flusssensors während des gewöhnlichen Beatmungsbetriebs ausgeführt werden, in welchem ein Patient durch die Beatmungsvorrichtung beatmet wird. Die Beatmung des Patienten durch die Beatmungsvorrichtung muss dafür nicht unterbrochen werden. Folglich ist die Kalibrations-Betriebssituation bevorzugt eine Betriebssituation in einem gewöhnlichen Beatmungsbetrieb, während welchem eine inspiratorische Kalibration des proximalen Atemgas-Flusssensors durchgeführt wird.

Zur Bereitstellung möglichst genauer Erfassungsergebnisse, den proximalen Atemgasfluss betreffend, kann gemäß einer vorteilhaften Weiterbildung der vorliegenden Erfindung wenigstens eine Vorrichtung aus Steuervorrichtung und Datenverarbeitungsvorrichtung dazu ausgebildet sein, eine Null-Kalibration der Erfassungswerte-Ausgabecharakteristik des proximalen Atemgas-Flusssensors zur Vermeidung oder Korrektur eines Driftfehlers durchzuführen. Die Null-Kalibration hat dabei die Wirkung, dass der proximale Atemgas-Flusssensor unmittelbar nach Durchführung der Null-Kalibration unter Erfassungsbedingungen, die einem Fehlen eines Gasflusses durch den proximalen Atemgas-Flusssensor entsprechen, einen innerhalb eines vorgegebenen Toleranzintervalls um einen Flusswert von Null gelegenen Erfassungswert ausgibt.

Bei dem bevorzugt als proximalen Atemgas-Flusssensor eingesetzten Differenzdrucksensor können zur Null-Kalibration die Drücke auf beiden Seiten des in jedem Differenzdrucksensor vorhandenen Strömungswiderstandsbauteils durch Betätigen entsprechender Ventile auf einen betragsmäßig gleichen Wert, vorzugsweise auf den Umgebungsdruck (Druck der Umgebungsatmosphäre), gesetzt werden. Dies ist eine Druckerfassungssituation, die einem völligen Fehlen eines Atemgasflusses durch den proximalen Atemgas-Flusssensor entspricht. In dieser Betriebssituation kann das Signal des proximalen Atemgas-Flusssensors durch Setzen auf Null null-kalibriert werden, sodass eine zwischenzeitlich möglicherweise eingesetzte Signaldrift wieder beseitigt wird oder das Signal erneut von dessen korrekten Koordinatenursprung aus zu verfälschen beginnt. Wird die Null-Kalibration, was bevorzugt der Fall ist, periodisch ausgeführt, etwa in vorbestimmten Zeitabschnitten oder nach einer vorbestimmten Anzahl an Atemhüben, so kann selbst im unvorteilhaftesten Betriebsfall, in welchem Umstände dauerhaft eine Signaldrift bewirken, sichergestellt werden, dass der bei einem Strömungsstillstand im proximalen Atemgas-Flusssensor erhaltene proximale Erfassungswert ausreichend nahe bei dem theoretischen Erfassungswert von Null liegt.

Da unmittelbar nach einer Null-Kalibration die Erfassungswerte-Ausgabecharakteristik des proximalen Atemgas-Flusssensors wenigstens hinsichtlich ihres Nulldurchgangs korrekt ist, ist die wenigstens eine Vorrichtung aus Steuervorrichtung und Datenverarbeitungsvorrichtung zu Erzielung nicht nur einer Erfassungswerte-Ausgabecharakteristik mit korrekten Nulldurchgang, sondern auch mit möglichst korrekten Werten bei einer Strömung von inspiratorischem Atemgas dazu ausgebildet, eine Quantifizierung des wenigstens einen inspiratorischen Fehlerparameters mit oder in einem von drei Atemhüben zu beginnen, welche auf eine Durchführung einer Null-Kalibration folgen. Bevorzugt erfolgt die Quantifizierung des wenigstens einen inspiratorischen Fehlerparameters unmittelbar nach der Null-Kalibration, also beispielsweise entweder noch im selben Atemhub, in welchem die Null-Kalibration durchgeführt wird oder in dem unmittelbar auf diesen folgenden Atemhub. Dann kann sichergestellt sein, dass die vom proximalen Atemgas-Flusssensor erhältlichen korrigierten proximalen inspiratorischen Erfassungswerte nicht nur nahe des Nulldurchgangs, sondern auch fern davon, bei inspiratorischem Atemgasfluss durch den proximalen Atemgas-Flusssensor ausreichend genau den wahren inspiratorischen Atemgasfluss wiedergeben.

Üblicherweise weist die Beatmungsvorrichtung im Exspirationspfad der Atemgasleitung ein Exspirationsventil auf, um Inspiration und Exspiration funktionell voneinander trennen zu können. Das Exspirationsventil, welches dazu ausgebildet ist, einen Atemgasfluss vom Patienten weg durchzulassen und zum Patienten hin zu sperren, kann zu diesem Zweck einen in eine Schließstellung vorgespannten Ventilkörper aufweisen, welcher durch einen exspiratorischen Atemgasfluss in Öffnungsrichtung von seinem Ventilsitz entfernt bzw. abgehoben werden kann. Dadurch kommt im herkömmlichen Beatmungsbetrieb der oben beschriebene Basisfluss zustande.

Für eine betragsmäßig möglichst genaue Quantifizierung des wenigstens einen inspiratorischen Fehlerparameters ist es hilfreich, den Basisfluss während Erfassungen des inspiratorischen Atemgasflusses zur Quantifizierung des wenigstens einen inspiratorischen Fehlerparameters zu unterbinden. Daher ist gemäß einer bevorzugten Weiterbildung die Steuervorrichtung dazu ausgebildet, das Exspirationsventil in der Kalibrations-Betriebssituation für die Erfassung des einen distalen inspiratorischen Atemgasfluss repräsentierenden Erfassungswerts des distalen Atemgas-Flusssensors und für die Erfassung des realen inspiratorischen Erfassungswerts des proximalen Atemgas-Flusssensors zu schließen oder/und, sofern das Exspirationsventil aus irgendeinem Grund bereits geschlossen sein sollte, geschlossen zu halten. Das Inspirationsventil kann hierzu einen durch die Steuervorrichtung betätigbaren Aktuator, etwa einen Stößel, aufweisen mit welchem der auf dem Ventilsitz ruhende und somit das Exspirationsventil verschließende Ventilkörper in Anlage an dem Ventilsitz gehalten werden kann.

Zur Einstellung des inspiratorischen Atemgases hinsichtlich gewünschter Gasfeuchte oder/und hinsichtlich einer gewünschten Verabreichungstemperatur oder/und zur Beimischung von Aerosolen, wie etwa von Aerosol-Medikamenten, zum inspiratorischen Atemgas kann die Beatmungsvorrichtung im Inspirationspfad der Atemgasleitung eine Konditioniervorrichtung zur Änderung der Temperatur oder/und der absoluten Feuchtigkeit des inspiratorischen Atemgases oder/und zur Beimischung von Aerosolen zum inspiratorischen Atemgas aufweisen.

Eine Veränderung der Temperatur oder eine Veränderung der absoluten Feuchtigkeit des inspiratorischen Atemgases oder eine Veränderung von beidem verändert in der Regel die Dichte des inspiratorischen Atemgases und damit das von einer vorgegebenen Atemgasmenge eingenommene Volumen. Somit hat eine Änderung der Temperatur oder der Feuchtigkeit des inspiratorischen Atemgases auf dem Weg vom distalen zum proximalen Atemgas-Flusssensor eine unmittelbare Auswirkung auf den erfassbaren Atemgasfluss. Gleiches gilt für eine Beimischung von Aerosolen zum Atemgas.

Die Konditioniervorrichtung ist dann, wenn sie vorhanden ist, bevorzugt in Strömungsrichtung des inspiratorischen Atemgases stromabwärts des distalen Atemgas-Flusssensors und stromaufwärts des proximalen Atemgas-Flusssensors angeordnet. Bevorzugt befindet sich die Konditioniervorrichtung wesentlich näher am distalen Atemgas-Flusssensor als am proximalen Atemgas-Flusssensor.

Daher ist die Steuervorrichtung bevorzugt dazu ausgebildet, in der Kalibrations-Betriebssituation den Betrieb der Konditioniervorrichtung zu beenden oder zu unterbrechen. Dadurch kann verhindert werden, dass sich die Feuchtigkeit oder/und die Temperatur des inspiratorischen Atemgases auf dem Weg vom distalen zum proximalen Atemgas-Flusssensor ändern oder die Änderung kann zumindest betragsmäßig verringert werden.

Bevorzugt weist die Beatmungsvorrichtung zur Ermittlung von Parameterwerten, welche für die Verwendung des ersten Modells benötigt werden, wenigstens einen distalen Temperatursensor zur Messung der distalen Temperatur des inspiratorischen Atemgasflusses im Bereich des distalen Atemgas-Flusssensors auf. Zusätzlich kann die Beatmungsvorrichtung einen proximalen Temperatursensor zur Messung der proximalen Temperatur des inspiratorischen Atemgasflusses im Bereich des proximalen Atemgas-Flusssensors aufweisen. Es kann jedoch alternativ die Körpertemperatur des beatmeten Patienten als proximale Temperatur des inspiratorischen Atemgasflusses angenommen werden.

Ebenso kann die Beatmungsvorrichtung zur Verwendung des ersten Modells wenigstens einen proximalen Drucksensor zur Messung des proximalen Drucks des inspiratorischen Atemgases im Bereich des proximalen Atemgas-Flusssensors aufweisen. Eine Möglichkeit zur Druckmessung am proximalen inspiratorischen Atemgasfluss ist bei Verwendung eines Differenzdrucksensors systemimmanent gegeben, da dieser den proximalen inspiratorischen Atemgasfluss über eine Druckdifferenz stromaufwärts und stromabwärts des Strömungswiderstandsbauteils ermittelt.

Ebenso kann die Beatmungsvorrichtung zur Verwendung des ersten Modells wenigstens einen distalen Drucksensor zur Messung des distalen Drucks des inspiratorischen Atemgases im Bereich des distalen Atemgas-Flusssensors aufweisen. Zusätzlich oder alternativ zu einem distalen Drucksensor kann die Beatmungsvorrichtung einen Drucksensor zur Messung des Drucks der Umgebungsatmosphäre des Beatmungsgeräts aufweisen. Dann etwa, wenn die Atemgas-Fördervorrichtung ein Gebläse umfasst, welches Umgebungsluft als inspiratorisches Atemgas ansaugt und vermittels der Atemgasleitung zum Patienten fördert, kann der Umgebungsdruck, obwohl er häufig betragsmäßig niedriger ist als der distale Druck des Atemgases näherungsweise als distaler Druck des Atemgases im Bereich des distalen Atemgas-Flusssensors verwendet werden. Dies gilt umso mehr, je näher der distale Atemgas-Flusssensor bei einem Ansaugort zum Ansaugen von Umgebungsluft aus der Umgebungsatmosphäre gelegen ist.

Weiterhin kann die Beatmungsvorrichtung einen distalen Feuchtigkeitssensor aufweisen zur Erfassung einer relativen oder absoluten Feuchtigkeit des distalen inspiratorischen Atemgases. Dieser distale Feuchtigkeitssensor kann im Bereich des distalen Atemgas-Flusssensors angeordnet sein. Dann, wenn Umgebungsluft als inspiratorisches Atemgas verwendet wird, kann der distale Feuchtigkeitssensor in einem Gehäuse der Beatmungsvorrichtung oder außerhalb desselben zur Erfassung der relativen oder absoluten Feuchtigkeit der Umgebungsluft angeordnet und ausgebildet sein, da sich die Feuchtigkeit der als inspiratorisches Atemgas angesaugten Umgebungsluft bis zum Erreichen des Anbringungsorts des distalen Atemgas-Flusssensors in der Regel nicht oder nur unwesentlich ändert.

Weiter alternativ kann eine bekannte Feuchtigkeit des inspiratorischen Atemgases durch eine Bedienperson über eine Eingabe/Ausgabevorrichtung in die Beatmungsvorrichtung eingegeben werden. Außerdem kann anstelle eines proximalen Feuchtigkeitssensors ausgehend von einer bekannten distalen Feuchtigkeit des inspiratorischen Atemgases die proximale Feuchtigkeit durch ein weiteres Modell auf Grundlage von Betriebsdaten, wie beispielsweise Heizleistung oder Betriebsleistung eines aktiven Befeuchters oder die Betriebsleistung eines Verneblers, durch die Datenverarbeitungsvorrichtung auf die Änderung der Feuchtigkeit des inspiratorischen Atemgases mittels der Konditioniervorrichtung geschlossen werden, ohne dass entsprechende Sensoren an der Beatmungsvorrichtung vorhanden sein müssten.

Bevorzugt übertragen alle Sensoren ihre Erfassungswerte zur Datenverarbeitungsvorrichtung, zur Datenspeicheranordnung oder zur Steuervorrichtung, wobei die Steuervorrichtung und die Datenverarbeitungsvorrichtung eine kombinierte Datenverarbeitungs- und Steuervorrichtung sein können oder gesonderte Vorrichtungen, welche datenübertragungsmäßig miteinander verbunden sind.

Zur Ermittlung eines Näherungswerts für den proximalen inspiratorischen Erfassungswert auf Grundlage des distalen inspiratorischen Erfassungswerts kann das erste Modell eine quantitative Änderung des inspiratorischen Atemgasflusses auf Grundlage einer Änderung der Temperatur berücksichtigen. Zusätzlich oder alternativ kann das erste Modell eine quantitative Änderung des inspiratorischen Atemgasflusses auf Grundlage einer Änderung des Drucks des Atemgases zwischen dem Anordnungsbereich, insbesondere dem Anordnungsort, des distalen Atemgas-Flusssensors und dem Anordnungsbereich, insbesondere dem Anordnungsort, des proximalen Atemgas-Flusssensors berücksichtigen. Eine solche quantitative Änderung des inspiratorischen Gasflusses auf Grundlage von Änderungen der Temperatur oder/und des Drucks können auf der idealen Gasgleichung beruhen.

Weiter zusätzlich oder alternativ kann das erste Modell eine quantitative Änderung des inspiratorischen Atemgasflusses auf Grundlage einer Änderung der Feuchtigkeit des Atemgases zwischen dem Anordnungsbereich, insbesondere dem Anordnungsort, des distalen Atemgas-Flusssensors und dem Anordnungsbereich, insbesondere dem Anordnungsort, des proximalen Atemgas-Flusssensors berücksichtigen.

Als Anordnungsbereich eines Atemgas-Flusssensors gilt dabei ein den Anordnungsort des betroffenen Atemgas-Flusssensors enthaltener örtlicher Bereich, dessen näher bei dem jeweils anderen Atemgas-Flusssensor gelegene Grenze wenigstens doppelt so weit von dem jeweils anderen Atemgas-Flusssensor entfernt ist wie von dem nähergelegenen betroffenen Atemgas-Flusssensor.

Auch die Atemgasleitung selbst kann aufgrund ihrer Elastizität Atemgas speichern, welches zunächst durch die Atemgas-Fördervorrichtung in der Atemgasleitung gefördert wird, aber aufgrund des Speichervermögens der elastischen Atemgasleitung den Patienten nicht erreicht. Daher kann das erste Modell auch eine quantitative Änderung des inspiratorischen Atemgasflusses auf Grundlage einer Elastizität der Atemgasleitung berücksichtigen.

Das erste Modell kann grundsätzlich ein Modell sein, welches auf empirisch bestimmten Kennlinien oder/und Kennfeldern oder/und Tabellen beruht, die vorab für eine Beatmungsvorrichtung oder ein bestimmtes Ausführungsmodell einer serienmäßig hergestellten Beatmungsvorrichtung im Labor ermittelt wurden. Die Datenverarbeitungsvorrichtung kann dann aus ermittelten Ausgangsgrößen, wie beispielsweise aus der distalen und der proximalen Atemgastemperatur, dem empirisch bestimmten ersten Modell die gewünschte Zielgröße, etwa die prozentuale Änderung des Atemgasflusses bezogen auf den distalen inspiratorischen Atemgasfluss, entnehmen. Zwischenwerte, beispielsweise der genannten Tabelle, können durch Interpolation erhalten werden.

Bevorzugt ist das erste Modell ein mathematisch-funktioneller Zusammenhang zwischen dem proximalen Atemgasfluss im Anordnungsbereich des proximalen Atemgas-Flusssensors und dem distalem Atemgasfluss im Anordnungsbereich des distalen Atemgas-Flusssensors, sodass die quantitative Änderung des inspiratorischen Atemgasflusses vom distalen Atemgas-Flusssensor zum proximalen Atemgas-Flusssensor durch analytische oder numerische Lösung eines Gleichungszusammenhangs ermittelbar ist.

Beispielsweise kann das erste Modell zu Berücksichtigung der Änderung des inspiratorischen Atemgasflusses aufgrund von Änderung der Temperatur oder/und des Druckes des Atemgases folgenden Term enthalten:

$$_{prox}^{pT}q(t)_{insp} = {}_{dist}q(t)_{insp} \cdot \frac{_{prox}T(t)_{insp}}{_{dist}T(t)_{insp}} \cdot \frac{_{dist}p(t)_{insp}}{_{prox}p(t)_{insp}} \qquad \text{Gl. 1}$$

Wobei $_{prox}^{pT}q_{insp}$ der auf einer Änderung von Druck oder/und Temperatur des Atemgases beruhende proximale inspiratorische Atemgasfluss zum Zeitpunkt t auf Grundlage des distalen inspiratorischen Atemgasflusses $_{dist}q(t)_{insp}$ zum Zeitpunkt t ist. T(t) bezeichnet die Temperatur des Atemgases zum Zeitpunkt t und p bezeichneten absoluten Druck des Atemgases zum Zeitpunkt t. Der vorangestellte tiefe Index gibt den Gültigkeitsort: proximal oder distal, des jeweiligen Werts an, der nachgestellte tiefe Index die Art des Atemgasflusses: inspiratorisch oder exspiratorisch.

Zur Berücksichtigung einer Änderung des inspiratorischen Atemgasflusses auf Grundlage einer Änderung seiner Feuchtigkeit kann das erste Modell folgenden Term enthalten:

$$_{prox}^{humid}q(t)_{insp} = {}^{humid}q(t) \qquad \text{Gl. 2}$$

Wobei $_{prox}^{humid}q(t)_{insp}$ der auf einer Änderung der Feuchtigkeit, insbesondere der absoluten Feuchtigkeit, des Atemgases beruhende proximale inspiratorische Atemgasfluss zum Zeitpunkt t ist. $^{humid}q(t)$ bezeichnet den zum Zeitpunkt t dem Atemgas zugeführten Fluss an Feuchtigkeit, etwa durch verdampftes oder vernebeltes Wasser oder durch ein verdampftes oder vernebeltes, ursprünglich flüssiges Medikament.

Zur Berücksichtigung einer Änderung des inspiratorischen Atemgasflusses auf Grundlage der Elastizität der Atemgasleitung kann das zweite Modell folgenden Term enthalten:

$$_{prox}^{tube}q(t)_{insp} = -^{tube}C \cdot \frac{d_{prox}p(t)_{insp}}{dt} \qquad \text{Gl. 3}$$

wobei $_{prox}^{tube}q(t)_{insp}$ der auf der Elastizität der Atemgasleitung beruhende proximale inspiratorische Atemgasfluss zum Zeitpunkt t ist. $^{tube}C$ bezeichnet die "Compliance", also den Kehrwert der Steifigkeit, der Atemgasleitung und $\frac{d_{prox}p(t)_{insp}}{dt}$ bezeichnet die Änderung des proximalen Drucks des inspiratorischen Atemgases mit der Zeit. Da bei einem positiven Druckgradienten die Elastizität der Atemgasleitung eine Verringerung des proximalen inspiratorischen Atemgasflusses bewirkt, ist der Term negativ.

Bevorzugt umfasst das erste Modell alle drei obigen Terme, sodass der unter Verwendung des ersten Modells ermittelte Näherungswert des proximalen inspiratorischen Atemgasflusses $_{prox}^{approx}q(t)_{insp}$ eine Summe der obigen Partikular-Flüsse darstellt:

$$_{prox}^{approx}q(t)_{insp} = _{prox}^{pT}q(t)_{insp} + _{prox}^{humid}q(t)_{insp} + _{prox}^{tube}q(t)_{insp} \qquad \text{Gl. 4}$$

oder ausgeschrieben:

$$_{prox}^{approx}q(t)_{insp} = _{dist}q(t)_{insp} \cdot \frac{_{prox}T(t)_{insp}}{_{dist}T(t)_{insp}} \cdot \frac{_{dist}p(t)_{insp}}{_{prox}p(t)_{insp}} + ^{humid}q(t) - ^{tube}C \cdot \frac{d_{prox}p(t)_{insp}}{dt}$$

$$\text{Gl. 5}$$

Für $^{humid}q(t)$ und $^{tube}C$ können für viele Betriebssituationen passende Anfangswerte in der Datenspeicheranordnung hinterlegt sein. Beispielsweise beträgt aufgrund empirischer Ermittlung $^{humid}q(t)$ in vielen Betriebssituationen etwa $0,04 \cdot {_{dist}q(t)_{insp}}$. Außerdem sind die an einer Beatmungsvorrichtung verwendeten Atemgasleitungen in der Regel bekannt, sodass auch deren Compliance gegenüber Druckänderungen in ihrem Inneren bekannt oder zumindest ermittelbar sind.

Bevorzugt ist die Beatmungsvorrichtung dazu ausgebildet, vor Beginn eines Beatmungsbetriebs einen Selbsttest durchzuführen, in dessen Verlauf beispielsweise die Compliance $^{tube}C$ der Atemgasleitung ermittelt werden kann und so auch Alterungseffekte der Atemgasleitung oder Veränderungen derselben durch lokale Umwicklung mit Klebeband und dergleichen berücksichtigt werden können. Auch zur Ermittlung der Compliance der Atemgasleitung ist es vorteilhaft, wenn die Steuervorrichtung dazu ausgebildet ist, das Exspirationsventil zu schließen und geschlossen zu halten, sodass einer Druckerhöhung in der Atemgasleitung eine Volumenerhöhung der Atemgasleitung zugeordnet werden kann. Die Volumenerhöhung kann im Selbsttest durch Integration der in die Atemgasleitung mit geschlossenem Exspirationsventil geförderten Atemgasflüsse über die Zeit erhalten werden.

Als Anfangswert kann eine Compliance der Atemgasleitung im Bereich von 1,8 bis 2,4 ml/hPa verwendet werden. Atemgasleitungen in Beatmungsvorrichtungen der Anmelderin weisen in der Regel eine Compliance von 2,0 bis 2,2 ml/hPa auf. Auch ein Wert aus diesem Wertebereich kann somit als Anfangswert der Compliance gesetzt sein.

Auch das zweite Modell, welches einen realen proximalen inspiratorischen Erfassungswert in einen theoretischen fehlerfreien und daher nahe beim wahren proximalen inspiratorischen Atemgasfluss liegenden proximalen inspiratorischen Erfassungswert umzurechnen gestattet, kann eine Schar von Kennlinien, Kennfeldern oder Tabellen sein, wobei dann der wenigstens eine qualifizierte inspiratorische Fehlerparameter als Scharparameter angeben kann, welche Kennlinie, welches Kennfeld oder welche Tabelle aus der Schar von Kennlinien, Kennfeldern oder Tabellen zur Korrektur von Erfassungswerten des proximalen Atemgas-Flusssensors heranzuziehen ist.

In der Praxis hat sich auch für das zweite Modell ein mathematisch-funktioneller, Zusammenhang zwischen einem realen inspiratorischen Erfassungswert des tatsächlich in der Atemgasleitung angeordneten proximalen Atemgas-Flusssensors und einem theoretischen inspiratorischen Erfassungswert bei hypothetischer Erfassung derselben Strömungssituation durch den hypothetischen, fehlerfrei arbeitenden proximalen Atemgas-Flusssensor als äußerst vorteilhaft handhabbar erwiesen. Wenngleich ein solches mathematisch-funktionelles Modell ein Modell beliebiger Ordnung sein kann, welches den realen inspiratorischen Erfassungswert mit dem theoretischen proximalen inspiratorischen Erfassungswert verknüpft, hat sich in der Praxis ein linearer Zusammenhang der beiden Erfassungswerte als ausreichend genau herausgestellt. Üblicherweise ist ein mathematisch-funktioneller Zusammenhang n-ter Ordnung durch n Koeffizienten parametrisiert. Dann sind n inspiratorische Fehlerparameter als die n Koeffizienten zu quantifizieren, um aus dem strukturellen mathematisch-funktionellen Zusammenhang eine eindeutige Gleichung zu formulieren, welche für einen betragsmäßig definierten Eingangswert einen betragsmäßig definierten Ausgangswert ausgibt.

Der Vorteil des bevorzugten linearen Zusammenhangs zwischen realem und theoretischem inspiratorischen Erfassungswert des proximalen Atemgas-Flusssensors besteht nicht nur darin, dass ein einziger Koeffizient zur Parametrisierung des linearen Zusammenhangs ausreicht und damit nur genau ein inspiratorischer Fehlerparameter zu quantifizieren ist. Der Vorteil besteht auch darin, dass dieser lineare Zusammenhang einfach nach dem inspiratorischen Fehlerparameter auflösbar ist und dieser so einfach und ausreichend genau analytisch ermittelbar sein kann. Beispielsweise kann das zweite Modell wie folgt als Gleichung dargestellt sein:

$$_{prox}^{theor}q(t)_{insp} = k_{insp} \cdot {}_{prox}^{real}q(t)_{insp} \qquad\qquad \text{Gl. 6}$$

wobei $_{prox}^{theor}q(t)_{insp}$ der theoretische Erfassungswert eines hypothetischen fehlerfrei arbeitenden proximalen Atemgas-Flusssensors zum Zeitpunkt t ist, wobei weiter $_{prox}^{real}q(t)_{insp}$ der reale Erfassungswert des tatsächlich in der Atemgasleitung angeordnete proximale Atemgas-Flusssensors zum Zeitpunkt t ist und wobei $k_{insp}$ der inspiratorische Fehlerparameter ist.

Die Datenverarbeitungsvorrichtung kann dazu ausgebildet sein, den wenigstens einen inspiratorischen Fehlerparameter durch ein Regressionsverfahren zu quantifizieren, um die Ermittlung des inspiratorischen Fehlerparameters auf mehrere Stützstellen zu stützen.

So kann in der Kalibrations-Betriebssituation für mehrere unterschiedliche Zeitpunkte jeweils der reale inspiratorische Erfassungswert erfasst und diesem ein auf Grundlage des distalen Erfassungswerts zu denselben Zeitpunkten unter Verwendung des ersten Modells ermittelter Näherungswert als der theoretische inspiratorische Erfassungswert zugeordnet werden:

$$_{prox}^{approx}q(t_i)_{insp} = k_{insp} \cdot {}_{prox}^{real}q(t_i)_{insp} \text{ mit i = 1, 2, 3, ...} \qquad\qquad \text{Gl. 7}$$

Der Wert $_{prox}^{approx}q(t_i)_{insp}$ kann einfach aus einer der Gleichungen 1, 4 oder 5 für jeden der Stütz-Zeitpunkte $t_i$ ermittelt werden. Die Anpassung des inspiratorischen Fehlerparameters $k_{insp}$ kann nach der Methode der kleinsten Fehlerquadrate erfolgen.

Je nach den Fehlereinflussquellen, welche den proximalen Atemgas-Flusssensor in der Kalibrations-Betriebssituation tatsächlich nachteilig beeinflussen, kann der angenommene bevorzugte lineare funktionelle Zusammenhang zwischen realem gemessenem Wert und idealem Näherungswert unterschiedlich gut passen.

Zur Beurteilung der Brauchbarkeit der erreichten Quantifizierung des wenigstens einen Fehlerparameters kann die Datenverarbeitungsvorrichtung dazu ausgebildet sein, einen die Anpassungsgüte der zur Quantifizierung des wenigstens einen inspiratorischen Fehlerparameters durchgeführten Regression repräsentierenden Gütewert zu ermitteln und mit einem vorbestimmten, eine Mindest-Güte repräsentierenden Güte-Schwellenwert zu vergleichen. Die Datenverarbeitungsvorrichtung ist dabei bevorzugt dazu ausgebildet, die Quantifizierung des wenigstens einen inspiratorischen Fehlerparameters zu verwerfen, wenn der Gütewert den Güte-Schwellenwert nicht wenigstens erreicht. Wegen des linearen Zusammenhangs kann als Gütewert für die dann erforderliche lineare Regression der bekannte R-Quadrat-Wert herangezogen werden. Dabei gibt ein Wert von 1 für R-Quadrat eine ideale Anpassung der Stützpunkte durch eine Gerade mit der Steigung $k_{insp}$ an und ein Wert von Null eine maximal untaugliche Anpassung. Beispielsweise kann die ermittelte Quantifizierung des inspiratorischen Fehlerparameters dann verworfen werden, wenn der Betrag von R-Quadrat für die zurückliegende lineare Regression kleiner als 0,75 ist.

Ebenso kann die Datenverarbeitungsvorrichtung dazu ausgebildet sein, die Quantifizierung des wenigstens einen inspiratorischen Fehlerparameters zu verwerfen, wenn der wenigstens eine inspiratorischen Fehlerparameter nicht wenigstens einen vorbestimmten inspiratorischen Mindest-Fehlerparameterwert erreicht. Ergibt die Quantifizierung des inspiratorischen Fehlerparameters beispielsweise nur eine Änderung des korrigierten proximalen inspiratorischen Erfassungswerts gegenüber dem realen proximalen inspiratorischen Erfassungswert von 10 %, kann die Quantifizierung des inspiratorischen Fehlerparameters verworfen werden und stattdessen mit dem bisher gültigen inspiratorischen Fehlerparameter oder mit den realen proximalen inspiratorischen Erfassungswerten weitergearbeitet werden. Beispielsweise kann der Mindest-Fehlerparameterwert einen Wert von zwischen 1,1 und 1,35, insbesondere von zwischen 1,2 und 1,35 aufweisen, vorzugsweise 1,3. Ist also der Betrag von $k_{insp}$ kleiner als der Mindest-Fehlerparameterwert, kann $k_{insp}$ verworfen werden.

Bisher wurde nur eine Korrektur eines Erfassungsfehlers des proximalen Atemgas-Flusssensors für den inspiratorischen Atemgasstroms diskutiert. Da ein und derselbe Atemgas-Flusssensor sowohl den inspiratorischen Atemgasstroms als auch den exspiratorischen Atemgasstroms quantitativ erfasst, liegt die Annahme nahe, das bei Auftreten fehlerhafter proximaler inspiratorischer Erfassungswerte auch die proximalen exspiratorischen Erfassungswerte fehlerhaft sind. Da jedoch der distale Atemgas-Flusssensor in der Regel nur zur Erfassung des inspiratorischen Atemgasflusses ausgebildet oder/und angeordnet ist, bedarf es einer weiteren Lösung, den proximalen Atemgas-Flusssensor auch hinsichtlich seiner Erfassung des exspiratorischen Atemgasflusses zu korrigieren.

Ein einfacher Ansatz kann sein, den inspiratorischen Fehlerparameter auch zur Korrektur von proximalen exspiratorischen Erfassungswerten zu verwenden. Allerdings haben Versuche gezeigt, dass gerade Differenzdruck-Flusssensoren als proximale Atemgas-Flusssensoren richtungsabhängig betragsmäßig unterschiedliche Fehler aufweisen können.

Die Kalibration des proximalen Atemgas-Flusssensors zur Korrektur seiner Erfassung eines exspiratorischen Atemgasstroms kann dadurch erreicht werden, dass in der Datenspeicheranordnung ein drittes Modell hinterlegt ist, welches als ein exspiratorisches Fehlermodell gestattet, für ein und dieselbe Betriebssituation einen Erfassungswert aus einem theoretischen exspiratorischen Erfassungswert, wie er von einem hypothetischen, fehlerfrei arbeitenden proximalen Atemgas-Flusssensor als Ergebnis einer Erfassung des proximalen exspiratorischen Atemgasflusses an die Datenverarbeitungsvorrichtung ausgegeben würde, und einem realen exspiratorischen Erfassungswert, wie er vom proximalen Atemgas-Flusssensor als Ergebnis einer Erfassung des proximalen exspiratorischen Atemgasflusses tatsächlich an die Datenverarbeitungsvorrichtung ausgegeben wird, unter Verwendung wenigstens eines exspiratorischen Fehlerparameters auf Grundlage des jeweils anderen Erfassungswerts zu ermitteln, wobei die Datenverarbeitungsvorrichtung dazu ausgebildet ist, auf Grundlage von den inspiratorischen Atemgasfluss repräsentierenden inspiratorischen Erfassungswerten des proximalen Atemgas-Flusssensors sowie auf Grundlage von den exspiratorischen Atemgasfluss repräsentierenden realen exspiratorischen Erfassungswerten des proximalen Atemgas-Flusssensors für wenigstens einen Atemhub ein dem Patienten verabreichtes inspiratorisches Atemgasvolumen und ein vom Patienten abgegebenes reales exspiratorisches Atemgasvolumen zu ermitteln,

wobei die Datenverarbeitungsvorrichtung weiter dazu ausgebildet ist, nach Maßgabe des dritten Modells den wenigstens einen exspiratorischen Fehlerparameter derart zu quantifizieren, dass ein betragsmäßiger Unterschied zwischen dem verabreichten inspiratorischen Atemgasvolumen und einem nach Maßgabe des dritten Modells ermittelten abgegebenen theoretischen exspiratorischen Atemgasvolumen innerhalb eines vorbestimmten Toleranzbereichs um den Wert Null gelegen ist,

wobei die Datenverarbeitungsvorrichtung noch weiter dazu ausgebildet ist, im weiteren Betrieb der Beatmungsvorrichtung den wenigstens einen quantifizierten exspiratorischen Fehlerparameter auf vom proximalen Atemgas-Flusssensor an die Datenverarbeitungsvorrichtung ausgegebene reale exspiratorische Erfassungswerte anzuwenden, um die tatsächlichen exspiratorischen Erfassungswerte des proximalen Atemgas-Flusssensors in betragsmäßig weniger stark fehlerbehaftete korrigierte exspiratorische Erfassungswerte umzuwandeln.

Mit anderen, möglicherweise einfacheren Worten: eine Korrektur der proximalen exspiratorischen Erfassungswerte eines proximalen Atemgas-Flusssensors kann auf Grundlage einer Asymmetrie in der Erfassung eines inspiratorischen Atemgasvolumens eines Atemhubs oder einer Anzahl von Atemhüben und der Erfassung eines exspiratorischen Atemgasvolumens für denselben Atemhub bzw. für dieselben Atemhübe erfolgen. Näherungsweise kann nämlich angenommen werden, dass, zwischen gleichen Anfangs- und Endzuständen des Patienten, das zwischen dem Anfangs- und dem Endzustand des Patienten in den Patienten eingeleitete Atemgasvolumen etwa dem vom Patienten abgegebenen Atemgasvolumen entspricht.

Die oben genannte Lösung zur Kalibration des Atemgas-Flusssensors zur Korrektur seiner exspiratorischen Erfassungswerte ist grundsätzlich von der Korrektur der inspiratorischen Erfassungswerte unabhängig, sodass sie nicht nur als Weiterbildung der bisher beschriebenen Beatmungsvorrichtung anwendbar ist, sondern als eigenständige Beatmungsvorrichtung. Die vorliegende Erfindung betrifft daher auch eine Beatmungsvorrichtung zur wenigstens unterstützenden künstlichen Beatmung eines Patienten, umfassend:

- eine Atemgasleitung zur Leitung von inspiratorischem Atemgas zu dem Patienten hin und zur Leitung von exspiratorischem Atemgas vom Patienten weg,
- eine Atemgas-Fördervorrichtung zur Förderung von inspiratorischem Atemgas in der Atemgasleitung,
- einen näher am patientenseitigen Ende der Atemgasleitung angeordneten proximalen Atemgas-Flusssensor zur quantitativen Erfassung eines inspiratorischen und eines exspiratorischen proximalen Atemgasflusses in der Atemgasleitung und zur Ausgabe entsprechender Erfassungswerte,
- eine Datenverarbeitungsvorrichtung, welche zur Verarbeitung von an die Datenverarbeitungsvorrichtung ausgegebenen Erfassungswerten des proximalen Atemgas-Flusssensors ausgebildet ist,
- eine mit der Datenverarbeitungsvorrichtung in Datenübertragungsverbindung stehende Datenspeicheranordnung,
- eine Steuervorrichtung zur Steuerung wenigstens der Atemgas-Fördervorrichtung und der Datenverarbeitungsvorrichtung,

wobei in der Datenspeicheranordnung ein drittes Modell hinterlegt ist, welches als ein exspiratorisches Fehlermodell gestattet, für ein und dieselbe Betriebssituation einen Erfassungswert aus einem theoretischen exspiratorischen Erfassungswert, wie er von einem hypothetischen, fehlerfrei arbeitenden proximalen Atemgas-Flusssensor als Ergebnis einer Erfassung des proximalen exspiratorischen Atemgasflusses an die Datenverarbeitungsvorrichtung ausgegeben würde, und einem realen exspiratorischen Erfassungswert, wie er vom proximalen Atemgas-Flusssensor als Ergebnis einer Erfassung des exspiratorischen proximalen Atemgasflusses tatsächlich an die Datenverarbeitungsvorrichtung ausgegeben wird, unter Verwendung wenigstens eines exspiratorischen Fehlerparameters auf Grundlage des jeweils anderen Erfassungswerts zu ermitteln, wobei die Datenverarbeitungsvorrichtung dazu ausgebildet ist, auf Grundlage von den inspiratorischen Atemgasfluss repräsentierenden inspiratorischen Erfassungswerten des proximalen Atemgas-Flusssensors sowie auf Grundlage von den exspiratorischen Atemgasfluss repräsentierenden realen exspiratorischen Erfassungswerten des proximalen Atemgas-Flusssensors für wenigstens einen Atemhub ein dem Patienten verabreichtes inspiratorisches Atemgasvolumen und ein vom Patienten abgegebenes reales exspiratorisches Atemgasvolumen zu ermitteln,
wobei die Datenverarbeitungsvorrichtung weiter dazu ausgebildet ist, nach Maßgabe des dritten Modells den wenigstens einen exspiratorischen Fehlerparameter derart zu quantifizieren, dass ein betragsmäßiger Unterschied zwischen dem verabreichten inspiratorischen Atemgasvolumen und einem nach Maßgabe des dritten Modells ermittelten abgegebenen theoretischen exspiratorischen Atemgasvolumen innerhalb eines vorbestimmten Toleranzbereichs um den Wert Null gelegen ist,
wobei die Datenverarbeitungsvorrichtung noch weiter dazu ausgebildet ist, im weiteren Betrieb der Beatmungsvorrichtung den wenigstens einen quantifizierten exspiratorischen Fehlerparameter auf vom proximalen Atemgas-Flusssensor an die Datenverarbeitungsvorrichtung ausgegebene reale exspiratorische Erfassungswerte anzuwenden, um die tatsächlichen exspiratorischen Erfassungswerte des proximalen Atemgas-Flusssensors in betragsmäßig weniger stark fehlerbehaftete korrigierte exspiratorische Erfassungswerte umzuwandeln.

Die nachfolgend erläuterte Weiterbildung der exspiratorischen Kalibration des proximalen Atemgas-Flusssensors zur Korrektur proximaler exspiratorischer Erfassungswerte gilt sowohl für eine Weiterbildung der eingangs beschriebenen Beatmungsvorrichtung, welche auch zur inspiratorischen Kalibration des proximalen Atemgas-Flusssensors hinsichtlich einer Korrektur proximaler inspiratorischer Erfassungswerte ausgebildet ist als auch für die im Absatz zuvor beschriebene Beatmungsvorrichtung, welche ohne die Möglichkeit zur inspiratorischen Kalibration des proximalen Atemgas-Flusssensors auskommt.

Unterschiedliche Werte für verabreichte inspiratorische Atemgasvolumina einerseits und für abgegebene exspiratorische Atemgasvolumina andererseits können unterschiedliche Ursachen haben. Daher kann die Datenverarbeitungsvorrichtung dazu ausgebildet sein, das über den wenigstens einen Atemhub hinweg ermittelte verabreichte inspiratorische Atemgasvolumen mit dem über denselben wenigstens einen Atemhub hinweg ermittelten abgegebenen exspiratorischen Atemgasvolumen zu vergleichen und abhängig von dem Vergleichsergebnis die Quantifizierung des wenigstens einen exspiratorischen Fehlerparameters auszuführen.

Zwar kann ein Atemgasvolumen, exspiratorisch wie inspiratorisch, lediglich eines Atemhubs zur Ausführung der exspiratorischen Kalibration ausreichen. Da jedoch Atemgasvolumina über unmittelbar aufeinanderfolgende Atemgashübe schwanken können, werden das inspiratorische Atemgasvolumen und das exspiratorische Atemgasvolumen bevorzugt über mehr als einen Atemhub hinweg ermittelt und optional durch die Anzahl der berücksichtigten Atemhübe geteilt, sodass ein mittleres inspiratorisches Atemgasvolumen und ein mittleres exspiratorisches Atemgasvolumen ermittelt werden. Eine vorteilhaft kurze Anzahl an, bevorzugt zusammenhängenden, Atemhüben, welche eine schnelle, aber ausreichend genaue Quantifizierung des exspiratorischen Fehlerparameters ermöglicht, sind drei Atemhübe. Es soll jedoch nicht ausgeschlossen sein, dass die genannten Atemgasvolumina über mehr oder weniger als drei Atemhübe hinweg ermittelt werden.

Das ermittelte verabreichte inspiratorische Atemgasvolumen kann auf Grundlage der realen inspiratorischen Erfassungswerte des proximalen Atemgas-Flusssensors ermittelt werden. Dann, wenn die Beatmungsvorrichtung auch zur zuvor beschriebenen inspiratorischen Kalibration und damit zur Korrektur proximaler inspiratorischer Erfassungswerte ausgebildet ist, kann die Datenverarbeitungsvorrichtung zur Erhöhung der Genauigkeit auch der exspiratorischen Kalibration dazu ausgebildet sein, das ermittelte verabreichte inspiratorische Atemgasvolumen auf Grundlage

a) von Näherungswerten für Erfassungswerte des proximalen Atemgas-Flusssensors nach Maßgabe des ersten Modells auf Grundlage von in der Betriebssituation erfassten Erfassungswerten des distalen Atemgas-Flusssensors oder
b) von korrigierten inspiratorischen Erfassungswerten des proximalen Atemgas-Flusssensors

zu ermitteln.

Wie oben bereits angedeutet wurde, kann ein betragsmäßiger Unterschied zwischen dem in gleichen Zeiträumen verabreichten inspiratorischen und abgegebenen exspiratorischen Atemgasvolumen mehrere Ursachen haben, von welchen möglicherweise nicht alle durch eine exspiratorische Kalibration ausgeblendet werden sollen. Beispielsweise kann die Ursache dafür, dass das in einer Zeitspanne verabreichte inspiratorische Atemgasvolumen betragsmäßig größer ist als das in derselben Zeitspanne abgegebene exspiratorische Atemgasvolumen, eine Leckage in der Atemgasleitung sein. Diese Leckage soll für Bedienpersonal weiterhin erkennbar und ermittelbar bleiben, sodass die Datenverarbeitungsvorrichtung dazu ausgebildet sein kann, dann, wenn das inspiratorische Atemgasvolumen betragsmäßig größer als das für ein und dieselbe Zeitspanne ermittelte exspiratorische Atemgasvolumen ist, eine Quantifizierung des exspiratorischen Fehlerparameters zu unterlassen. Auch andere technische Folgen als das bloße Unterlassen einer Quantifizierung des exspiratorischen Fehlerparameters können technisch sinnvoll sein.

Grundsätzlich kann die Datenverarbeitungsvorrichtung gemäß einer Weiterbildung der vorliegenden Erfindung dazu ausgebildet sein, nach der Ermittlung des verabreichten inspiratorischen Atemgasvolumens und des vom Patienten im selben Zeitraum abgegebenen realen exspiratorischen Atemgasvolumens die beiden Atemgasvolumina betragsmäßig zu vergleichen und abhängig vom Vergleichsergebnis wenigstens eine der folgenden Maßnahmen auszuführen:

i) dann, wenn das verabreichte inspiratorische Atemgasvolumen betragsmäßig größer ist als das im selben Zeitraum abgegebene reale exspiratorische Atemgasvolumen, den wenigstens einen exspiratorischen Fehlerparameter zu quantifizieren, indem er betragsmäßig gleich dem wenigstens einen inspiratorischen Fehlerparameter gesetzt wird,
ii) dann, wenn das verabreichte inspiratorische Atemgasvolumen um ein vorbestimmtes Maß betragsmäßig größer ist als das abgegebene exspiratorische Atemgasvolumen, sowohl den wenigstens einen exspiratorischen Fehlerparameter als auch den wenigstens einen inspiratorischen Fehlerparameter auf den für den jeweiligen Parameter zuletzt gültigen Wert zu setzen,
iii) dann, wenn das verabreichte inspiratorische Atemgasvolumen betragsmäßig kleiner ist als das abgegebene reale exspiratorische Atemgasvolumen, nach Maßgabe des dritten Modells den wenigstens einen exspiratorischen Fehlerparameter derart zu quantifizieren, dass ein betragsmäßiger Unterschied zwischen dem verabreichten inspiratorischen Atemgasvolumen und einem nach Maßgabe des dritten Modells ermittelten abgegebenen theoretischen exspiratorischen Atemgasvolumen innerhalb eines vorbestimmten Toleranzbereichs um den Wert Null gelegen ist.

Ein sinnvoller Toleranzbereich kann etwa zwischen 3 und 8 % des an der Beatmungsvorrichtung zur Verabreichung als Soll-Volumen eingestellten Tidalvolumens sein. Vorzugsweise beträgt der Toleranzbereich 5 % des Tidalvolumens.

Für das dritte Modell gilt das oben zum zweiten Modell Gesagte. Bevorzugt ist also auch das dritte Modell ein linearer Zusammenhang zwischen dem an die Datenverarbeitungsvorrichtung ausgegebenen realen proximalen exspiratorischen Erfassungswert und dem theoretischen proximalen exspiratorischen Erfassungswert eines hypothetischen fehlerfrei arbeitenden proximalen Atemgas-Flusssensors. Die nachfolgende Gleichung gibt ein Beispiel für ein mögliches lineares drittes Modell an:

$$\genfrac{}{}{0pt}{}{theor}{prox}q(t)_{exsp} = k_{exsp} \cdot \genfrac{}{}{0pt}{}{real}{prox}q(t)_{exsp} \qquad \text{Gl. 8}$$

Die Datenverarbeitungsvorrichtung kann weiter zur Durchführung einer Plausibilitätsüberprüfung der inspiratorischen und der exspiratorischen Kalibration ausgebildet sein. Zur Plausibilitätsüberprüfung der inspiratorischen Kalibration kann die Datenverarbeitungsvorrichtung dazu ausgebildet sein zu überprüfen, vorzugsweise fortwährend zu überprüfen, ob über wenigstens einen Atemhub hinweg, vorzugsweise über mehrere unmittelbar aufeinanderfolgende Atemhübe hinweg, der Unterschiedsbetrag zwischen einem aufgrund von realen inspiratorischen Atemgasflüssen ermittelten realen verabreichten inspiratorischen Atemgasvolumen und einem aufgrund von korrigierten inspiratorischen Atemgasflüssen ermittelten korrigierten verabreichten inspiratorischen Atemgasvolumen kleiner als ein vorbestimmter inspiratorischer Plausibilitätstoleranzwert ist.

Der inspiratorische Plausibilitätstoleranzwert ist vorzugsweise kleiner als die prozentuale Fehlertoleranz des verwendeten proximalen Atemgas-Flusssensors, multipliziert mit dem als Soll-Wert eingestellten Tidalvolumen. Hierdurch kann eine vorübergehend sinnvolle Korrektur inspiratorischer Atemgasflüsse wieder automatisiert rückgängig gemacht werden, etwa wenn sich ein das Strömungswiderstandsbauteil ursprünglich behindernder Flüssigkeitstropfen vom Strömungswiderstandsbauteil gelöst hat und dieses nun nicht mehr behindert.

Vorzugsweise beträgt der inspiratorische Plausibilitätstoleranzwert einen Bruchteil, vorzugsweise den halben Wert, der prozentualen Fehlertoleranz des proximalen Atemgas-Flusssensors, multipliziert mit dem als Soll-Wert eingestellten Tidalvolumen. Alternativ kann ein vorbestimmter absoluter Volumenwert als inspiratorischer Plausibilitätstoleranzwert angenommen werden, etwa ein Wert im Bereich von 3 bis 8 ml, insbesondere ein Wert von 5 ml. Abhängig vom eingestellten Tidalvolumen kann der größere von beiden Werten aus einem absoluten Volumenwert und dem mit dem eingestellten Tidalvolumen multiplizierten vorbestimmten Bruchteil der maximalen prozentualen Fehlertoleranz des Atemgas-Flusssensors als der inspiratorische Plausibilitätstoleranzwert verwendet werden.

Ergibt die Plausibilitätsüberprüfung keine ausreichende Plausibilität der Korrektur der proximalen inspiratorischen Erfassungswerte, kann die Datenverarbeitungsvorrichtung dazu ausgebildet sein, die Korrektur zurückzunehmen, also etwa den wenigstens inspiratorischen Fehlerparameter auf 1 zu setzen.

Entsprechendes gilt mutatis mutandis für eine Plausibilitätsüberprüfung der exspiratorischen Kalibration, also eine Überprüfung des ermittelten wenigstens einen exspiratorischen Fehlerparameters. Dies kann wie die Plausibilitätsüberprüfung der inspiratorischen Kalibration erfolgen, mit der Maßgabe, dass statt des verabreichten inspiratorischen Atemgasvolumens ein abgegebenes exspiratorisches Atemgasvolumen ermittelt wird. Im Rahmen der Plausibilitätsüberprüfung kann die Datenverarbeitungsvorrichtung prüfen, ob der Unterschiedsbetrag zwischen einem auf Grundlage realer proximaler exspiratorischer Atemgasflüsse ermittelten realen exspiratorischen Atemgasvolumen und einem auf Grundlage korrigierter proximaler exspiratorischer Atemgasflüsse ermittelten korrigierten exspiratorischen Atemgasvolumen kleiner ist als ein vorbestimmter erster exspiratorischer Plausibilitätstoleranzwert.

Zusätzlich oder alternativ kann die Datenverarbeitungsvorrichtung zur Durchführung einer Plausibilitätsüberprüfung der exspiratorischen Kalibration prüfen, ob der Betrag eines Unterschieds zwischen einem über mehrere, vorzugsweise mehrere zusammenhängende, Atemhübe ermittelten mittleren realen verabreichten proximalen exspiratorischen Atemgasvolumen und einem über mehrere, vorzugsweise mehrere zusammenhängende, Atemhübe ermittelten mittleren realen verabreichten proximalen inspiratorischen Atemgasvolumen betragsmäßig um mehr als einen vorbestimmten zweiten exspiratorischen Plausibilitätstoleranzwert von einem Betrag eines Unterschieds zwischen einem über mehrere, vorzugsweise mehrere zusammenhängende, Atemhübe ermittelten mittleren korrigierten verabreichten proximalen exspiratorischen Atemgasvolumen und einem über mehrere, vorzugsweise mehrere zusammenhängende, Atemhübe ermittelten mittleren korrigierten verabreichten proximalen inspiratorischen Atemgasvolumen abweicht.

Wiederum kann der erste oder/und der zweite exspiratorische Plausibilitätstoleranzwert ein vorbestimmter absoluter Volumenwert sein, wie etwa ein Wert zwischen 3 bis 8 ml, vorzugsweise 5 ml, oder/und kann ein auf Grundlage des während der Erfassung der für die Plausibilitätsüberprüfung verwendeten Atemgasvolumina eingestellten Tidalvolumens und einer prozentualen Fehlertoleranz, insbesondere einer maximalen prozentualen Fehlertoleranz, des proximalen Atemgas-Flusssensors berechneter exspiratorischer Plausibilitätstoleranzwert sein. Bevorzugt ist auch der exspiratorische Plausibilitätstoleranzwert ein mit einem Bruchteil der prozentualen Fehlertoleranz des proximalen Atemgas-Flusssensors multipliziertes Tidalvolumen.

Vorzugsweise sind der erste und der zweite exspiratorische Plausibilitätstoleranzwert identisch. Dies muss jedoch nicht so sein.

Ergibt die Plausibilitätsüberprüfung keine ausreichende Plausibilität der Korrektur der proximalen exspiratorischen Erfassungswerte, kann die Datenverarbeitungsvorrichtung dazu ausgebildet sein, die Korrektur zurückzunehmen, also etwa den wenigstens exspiratorischen Fehlerparameter auf 1 zu setzen.

Die vorliegende Erfindung wird nachfolgend anhand der beiliegenden Zeichnungen näher erläutert werden. Es stellt dar:

Fig. 1   eine Ausführungsform einer erfindungsgemäßen Beatmungsvorrichtung,

Fig. 2   eine grafische Darstellung von inspiratorischen Atemgasvolumina, exspiratorischen Atemgasvolumina, eingestellten Tidalvolumina und eines Toleranzbereichs um die Tidalvolumina, für zahlreiche aufeinanderfolgende Atemhübe,

Fig. 3   eine grafische Darstellung während eines Beatmungsbetriebs der Beatmungsvorrichtung von Figur 1 einem Patienten verabreichten und von diesem abgegebenen Atemgasflüssen während einer inspiratorischen und einer exspiratorischen Kalibration,

Fig. 4 eine beispielhafte grafische Darstellung eines inspiratorischen Beatmungsbetriebs der Beatmungsvorrichtung von Figur 1 mit tatsächlich messtechnisch erfassten distalen und proximalen realen inspiratorischen Atemgasflusswerten, mit gemäß dem ersten Modell angenäherten proximalen inspiratorischen Atemgasflusswerten und mit gemäß dem zweiten Modell ermittelten kalibrierten theoretischen Atemgasflusswerten, wie sie ein hypothetisch fehlerlos arbeitender Flusssensor ermitteln würde, und

Fig. 5 eine beispielhafte grafische Darstellung der Bestimmung des inspiratorischen Fehlerparameters $k_{insp}$ durch lineare Regression anhand der in Figur 4 gezeigten Daten des inspiratorischen Beatmungsbetriebs.

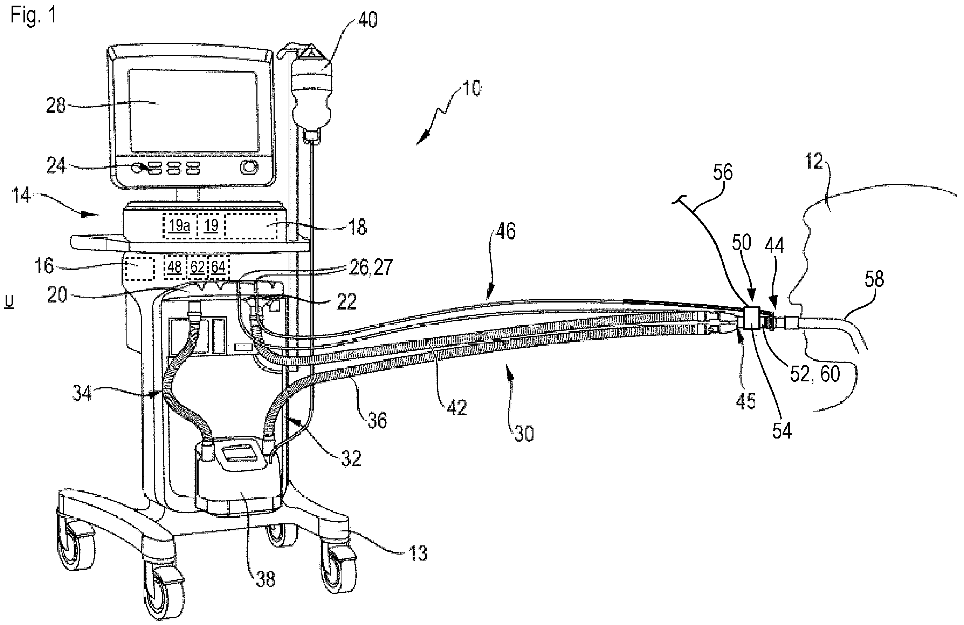

In Figur 1 ist eine erfindungsgemäße Ausführungsform einer Beatmungsvorrichtung allgemein mit 10 bezeichnet. Die Beatmungsvorrichtung 10 dient im dargestellten Beispiel zur künstlichen Beatmung eines humanen Patienten 12.

Die Beatmungsvorrichtung 10 kann als mobile Beatmungsvorrichtung 10 auf einem rollbaren Gestell 13 aufgenommen sein.

Die Beatmungsvorrichtung 10 weist ein Gehäuse 14 auf, in dem - von außen wegen des blickdichten Gehäusematerials nicht erkennbar - eine Atemgas-Fördervorrichtung 16, eine Steuervorrichtung 18 und eine Datenverarbeitungsvorrichtung 19 mit einer Datenspeicheranordnung 19a aufgenommen sind.

Die Atemgas-Fördervorrichtung 16 ist in an sich bekannter Weise aufgebaut und weist im dargestellten Beispiel eine Pumpe oder einen Verdichter oder ein Gebläse auf, die jeweils lastveränderlich durch die Steuervorrichtung 18 ansteuerbar sind und daher nicht nur der Einleitung von Atemgas aus der Umgebungsatmosphäre U als einer Atemgasquelle in die Beatmungsvorrichtung 10, sondern auch der Änderung des Drucks des eingeleiteten Atemgases dienen. Die Atemgasquelle kann alternativ auch durch einen Druckbehälter gebildet sein, welcher an das Gehäuse 14 der Beatmungsvorrichtung 10 anschließbar ist. Die Atemgas-Fördervorrichtung 16 kann, insbesondere im Falle eines unter Druck stehenden Gasvorrats als Atemgasquelle ein Reduzierventil und dergleichen aufweisen. Weiter weist die Beatmungsvorrichtung 10 in an sich bekannter Weise ein Inspirationsventil 20 und ein Exspirationsventil 22 auf.

Die Steuervorrichtung 18 und die Datenverarbeitungsvorrichtung 19 sind üblicherweise als Computer oder Mikroprozessor realisiert. Die Steuervorrichtung 18 und die Datenverarbeitungsvorrichtung 19 sind mit der Datenspeicheranordnung 19a datenübertragungsmäßig verbunden, in welcher für den Betrieb der Beatmungsvorrichtung 10 notwendige Daten gespeichert und, etwa als Sensor-Erfassungswerte speicherbar sind. Die Datenspeicheranordnung 19a kann bei Netzwerkbetrieb auch außerhalb des Gehäuses 14 gelegen und durch eine Datenübertragungsverbindung mit der Steuervorrichtung 18 und der Datenverarbeitungsvorrichtung 19 verbunden sein. Die Datenübertragungsverbindung kann durch eine Kabel- oder eine Funkstrecke gebildet sein. Um jedoch zu verhindern, dass Störungen der Datenübertragungsverbindung sich auf den Betrieb der Beatmungsvorrichtung 10 auswirken können, ist die Datenspeicheranordnung 19a bevorzugt in die Steuervorrichtung 18 oder in die Datenverarbeitungsvorrichtung 19 integriert oder wenigstens im selben Gehäuse 14 wie diese aufgenommen. Die Steuervorrichtung 18 und die Datenverarbeitungsvorrichtung 19 können durch eine einzige Vorrichtung realisiert sein.

Zur Eingabe von Daten in die Beatmungsvorrichtung 10 bzw. genauer in die Steuervorrichtung 18 und die Datenverarbeitungsvorrichtung 19 weist die Beatmungsvorrichtung 10 einen Dateneingabevorrichtung 24 auf, welche in dem in Figur 1 dargestellten Beispiel durch eine Tastatur repräsentiert ist. Die Steuervorrichtung 18 und die Datenverarbeitungsvorrichtung 19 können alternativ oder zusätzlich zur dargestellten Tastatur Daten über verschiedene Dateneingänge erhalten, etwa über eine Netzwerkleitung, eine Funkstrecke oder über Sensoranschlüsse 26, auf die weiter unten im Einzelnen eingegangen wird.

Zur Ausgabe von Daten an den behandelnden Therapeuten kann die Beatmungsvorrichtung 10 eine Ausgabevorrichtung 28 aufweisen, im dargestellten Beispiel einen Bildschirm.

Zur künstlichen Beatmung ist der Patient 12 mit der Beatmungsvorrichtung 10, genauer mit der Atemgas-Fördervorrichtung 16 im Gehäuse 14, über eine Atemgasleitung 30 verbunden. Der Patient 12 ist hierfür mit einem endotrachealen Rohr 58 intubiert. Anstelle des endotrachealen Rohr 58 kann eine auf den Mund-, den Nasen- oder den Mund-und-Nasenbereich des Patienten 12 aufgesetzte Maske als Schnittstelle zwischen der Beatmungsvorrichtung 10 und dem Patienten 12 verwendet sein.

Die Atemgasleitung 30, über welche inspiratorisches Atemgas mittels der Atemgas-Fördervorrichtung 16 aus der Umgebungsatmosphäre U in die Lunge des Patienten 12 gefördert werden kann, weist außerhalb des Gehäuses 14 einen Inspirationsschlauch 32 auf. Der Inspirationsschlauch 32 kann unterbrochen sein und einen ersten Inspirationsteilschlauch 34 und einen zweiten Inspirationsteilschlauch 36 aufweisen, zwischen welchen eine Konditionierungsvorrichtung 38 zur gezielten Befeuchtung und Temperierung des dem Patienten 12 zugeführten frischen Atemgases vorgesehen sein kann. Die Konditionierungsvorrichtung 38 kann mit einem externen Flüssigkeitsvorrat 40 verbunden sein, über den Wasser zur Befeuchtung oder auch ein Aerosol-Medikament, etwa zur Entzündungshemmung oder zur Erweiterung der Atemwege, dem inspiratorischen Atemgas zugeführt werden kann. Bei einem Einsatz der vorliegenden Beatmungsvorrichtung 10 als Anästhesie-Beatmungsvorrichtung können auf diese Weise volatile Anästhetika kontrolliert über die Beatmungsvorrichtung 10 an den Patienten 12 abgegeben werden. Die Konditionierungsvorrichtung 38 sorgt dafür, dass das inspiratorische Atemgas dem Patienten 12 mit einem vorbestimmten Feuchtegehalt, gegebenenfalls unter Zugabe eines Medikamenten-Aerosols, und mit einer vorbestimmten Temperatur zugeleitet wird.

Die Atemgasleitung 30 weist neben dem bereits erwähnten Inspirationsventil 20 das Exspirationsventil 22 und weiter einen Exspirationsschlauch 42 auf, über welchen verstoffwechseltes exspiratorisches Atemgas aus der Lunge des Patienten 12 in die Umgebungsatmosphäre U abgeblasen wird.

Der Inspirationsschlauch 32 ist mit dem Inspirationsventil 20 gekoppelt, der Exspirationsschlauch 42 mit dem Exspirationsventil 22. Von den beiden Ventilen ist jeweils nur eines gleichzeitig zum Durchlass einer Gasströmung geöffnet. Die Betätigungssteuerung der Ventile 20 und 22 erfolgt durch die Steuervorrichtung 18.

Während eines Beatmungszyklus ist zunächst für die Dauer der Inspirationsphase das Exspirationsventil 22 geschlossen und das Inspirationsventil 20 geöffnet, sodass inspiratorisches Atemgas vom Gehäuse 14 zum Patienten 12 geleitet werden kann. Eine Strömung des inspiratorischen Atemgases wird durch gezielte Druckerhöhung des Atemgases durch die Atemgas-Fördervorrichtung 16 bewirkt. Aufgrund der Druckerhöhung strömt das inspiratorische Atemgas in die Lunge des Patienten 12 und expandiert dort den lungennahen Körperbereich, also insbesondere den Brustkorb, gegen die individuelle Elastizität der lungennahen Körperteile. Hierdurch steigt auch der Gasdruck im Inneren der Lunge des Patienten 12 an.

Am Ende der Inspirationsphase wird das Inspirationsventil 20 geschlossen und das Exspirationsventil 22 geöffnet. Es beginnt die Exspirationsphase. Aufgrund des bis zum Ende der Inspirationsphase erhöhten Gasdrucks des in der Lunge des Patienten 12 befindlichen Atemgases strömt dieses nach dem Öffnen des Exspirationsventils 22 in die Atmosphäre, wobei sich mit fortschreitender Strömungsdauer der Gasdruck in der Lunge des Patienten 12 verringert. Erreicht der Gasdruck in der Lunge 12 einen an der Beatmungsvorrichtung 10 eingestellten positiven end-exspiratorischen Druck, also einen geringfügig höheren Druck als den Atmosphärendruck, wird die Exspirationsphase mit dem Schließen des Exspirationsventils 22 beendet und es schließt sich ein weiterer Beatmungszyklus an. Genauer wird der geringfügig höhere Druck gehalten, bis der Patient triggert oder eine vorbestimmte Haltedauer erreicht ist und die Beatmungsvorrichtung den weiteren Beatmungszyklus beginnt.

Während der Inspirationsphase wird dem Patienten 12 das sogenannte Tidalvolumen zugeführt, also das als Soll-Volumen eingestellte Atemgas-Volumen pro Atemhub. Das Tidalvolumen multipliziert mit der Anzahl an Beatmungszyklen pro Minute, also multipliziert mit der Beatmungsfrequenz, ergibt das Minutenvolumen der vorliegend durchgeführten künstlichen Beatmung.

Bevorzugt ist die Beatmungsvorrichtung 10, insbesondere die Steuervorrichtung 18 und die Datenverarbeitungsvorrichtung 19, dazu ausgebildet, Beatmungs-Betriebsparameter, die den Beatmungsbetrieb der Beatmungsvorrichtung 10 kennzeichnen, während des Beatmungsbetriebs wiederholt zu aktualisieren bzw. zu ermitteln, um sicherzustellen, dass der Beatmungsbetrieb zu jedem Zeitpunkt möglichst optimal auf den jeweils zu beatmenden Patienten 12 abgestimmt ist. Besonders vorteilhaft erfolgt die Bestimmung eines oder mehrerer Beatmungs-Betriebsparameter mit der Beatmungsfrequenz, sodass für jeden Beatmungszyklus aktuelle und damit optimal an den Patienten 12 angepasste Beatmungs-Betriebsparameter bereitgestellt werden können.

Hierzu ist die Beatmungsvorrichtung 10 mit einem oder mehreren Sensoren datenübertragungsmäßig verbunden, welche den Zustand des Patienten oder/und den Betrieb der Beatmungsvorrichtung überwachen.

Einer dieser Sensoren ist ein proximaler Atemgas-Flusssensor 44, welcher auf der dem Patienten 12 näher gelegenen Seite eines Y-Verbindungsstücks 45 angeordnet ist und dort den in der Atemgasleitung 30 herrschenden Atemgas-Fluss erfasst. Der Atemgas-Flusssensor 44 ist mittels einer Sensor-Leitungsanordnung 46 mit den Dateneingängen 26 der Steuervorrichtung 18 gekoppelt. Die Sensor-Leitungsanordnung 46 kann, muss jedoch nicht, elektrische Signalübertragungsleitungen umfassen. Sie kann ebenso Schlauchleitungen aufweisen, die den in Strömungsrichtung beiderseits des Atemgas-Flusssensors 44 herrschenden Gasdruck an die Dateneingänge 26 übertragen, wo diese von Drucksensoren 27 quantifiziert werden. Die Drucksensoren 27 sind dann Teil des Atemgas-Flusssensors 44. Der Atemgas-Flusssensor 44 ist zwar bevorzugt ein nach dem Differenzdruck-Prinzip arbeitender Atemgas-Flusssensor, kann jedoch auch ein nach einem anderen physikalischen Wirkprinzip arbeitender Atemgas-Flusssensor sein.

Im Gehäuse 14 ist ein weiterer Atemgas-Flusssensor 48 vorgesehen, welcher aufgrund seiner größeren Entfernung vom Patienten 12 - verglichen mit dem proximalen Atemgas-Flusssensor 44 - als distaler Atemgas-Flusssensor 48 bezeichnet ist. Der distale Atemgas-Flusssensor 48 kann ebenfalls ein Differenzdruck-Flusssensor sein, ist jedoch bevorzugt ein thermischer Flusssensor, wie etwa ein Hitzdrahtanemometer.

In der Atemgasleitung 30 zwischen dem Y-Verbindungsstück 45 und dem Atemgas-Flusssensor 44 kann eine weitere Sensoranordnung 50 angeordnet sein, umfassend ein als Messküvette 52 ausgebildetes Gehäuse 52 und eine Detektorbaugruppe 54, um sowohl im exspiratorischen wie auch im inspiratorischen Atemgas-Hauptstrom den Sauerstoffgehalt oder/und durch NDIR den Kohlendioxidgehalt des Atemgases zu erfassen. Die Sensoranordnung 50 ist über eine Signalleitung 56 mit der Steuervorrichtung 18 und der Datenverarbeitungsvorrichtung gekoppelt und überträgt an diese die Erfassungsergebnisse ihrer Detektorbaugruppe 54 zur weiteren Auswertung. In der weiteren Sensoranordnung 50 ist außerdem ein Temperatursensor 60 zur Erfassung der proximalen Temperatur des Atemgases im Anordnungsbereich des proximalen Atemgas-Flusssensors 44 angeordnet.

Beim distalen Atemgas-Flusssensor 48 sind ein distaler Temperatursensor 62 und ein distaler Drucksensor 64 angeordnet, um die distale Temperatur bzw. den distalen Druck des Atemgases im Anordnungsbereich des distalen Atemgas-Flusssensors 48 zu erfassen. Die Drucksensoren 27, welche die über die Sensor-Leitungsanordnung 46 gelieferten Drücke im proximalen Atemgas-Flusssensor 44 erfassen, erfassen somit den proximalen Druck des Atemgases im Anordnungsbereich des proximalen Atemgas-Flusssensors 44.

Der proximale Atemgas-Flusssensor 44, einschließlich der Drucksensoren 27, der distale Atemgas-Flusssensor 48, der proximale Temperatursensor 60, der distale Temperatursensor 62 und der distale Drucksensor 64 übertragen ihre Erfassungssignale an die Datenverarbeitungsvorrichtung 19 bzw. an die Datenspeicheranordnung 19a.

Darüber hinaus kann die Steuervorrichtung 18 Betriebsdaten wie eine von der Konditioniervorrichtung 38 abgerufene Heizleistung oder/und eine von der Konditioniervorrichtung 38 abgerufene Leistung eines in die Konditioniervorrichtung 38 eingebauten Verneblers oder/und Verdampfers als ein Befeuchter an die Datenverarbeitungsvorrichtung 19 bzw. an die Datenspeicheranordnung 19a übertragen. In der Datenspeicheranordnung 19a ist ein Berechnungsmodell gespeichert, welches es der Datenverarbeitungsvorrichtung 19 ermöglicht, anhand der Betriebsdaten der Konditioniervorrichtung 38 den Volumenstrom an Wasser- oder allgemein Feuchtigkeitsdampf näherungsweise zu ermitteln, welcher dem inspiratorischen Atemgas stromabwärts des distalen Atemgas-Flusssensors 48 zugeführt wird.

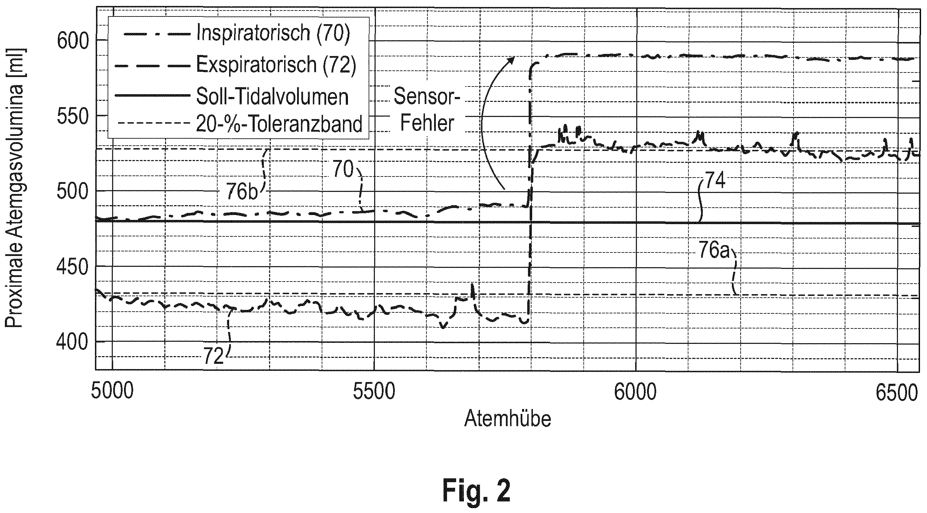

In Figur 2 ist eine graphische Datenaufzeichnung eines Beatmungsvorgangs dargestellt. Die Abszissenachse zeigt aufsteigend die aufeinanderfolgenden Atemhübe, die Ordinatenachse zeigt dem jeweiligen Atemhub zugeordnet je einen realen proximalen Erfassungswert für ein inspiratorisches Atemgasvolumen, einen realen proximalen Erfassungswert für ein exspiratorisches Atemgasvolumen, ein zu verabreichendes Soll-Tidalvolumen und ein Toleranzband um den Wert des Soll-Tidalvolumens von jeweils beiderseits 10 % bezogen auf das Soll-Tidalvolumen. Es besteht also ein Toleranzband mit einer Breite von 20 % des Soll-Tidalvolumens, in dessen Mitte das Soll-Tidalvolumen von im vorliegenden Fall 480 ml liegt. Die proximalen Erfassungswerte der inspiratorischen und exspiratorischen Atemgasflüsse werden vom proximalen Atemgas-Flusssensor 44 erfasst und an die Datenverarbeitungsvorrichtung 19 bzw. die Datenspeicheranordnung 19a übertragen. Diese integriert die Atemgasflüsse für jeden Atemhub über dessen Atemhubdauer zu den dargestellten Atemgasvolumina auf.

Der Graph 70 zeigt die realen proximalen inspiratorischen Atemgasvolumina, der Graph 72 die realen proximalen exspiratorischen Atemgasvolumina und der Graph 74 das Soll-Tidalvolumen. Mit gestrichelten Linien 76a und 76b sind die Untergrenze und die Obergrenze des Toleranzbandes um das Soll-Tidalvolumen bezeichnet.

Da sich der Wert des Soll-Tidalvolumens im Verlauf des in Figur 2 gezeigten Ausschnitts des Beatmungsvorgangs nicht ändert, ist der Graph 74 des Soll-Tidalvolumens eine zur Abszisse parallele Gerade.

Etwa bis zum Atemhub Nr. 5699 verlaufen die Werte des erfassten realen proximalen inspiratorischen Atemgasflusses mit einer leichten Drift im Verlauf der Beatmung hin zu betragsmäßig größer werdenden Erfassungswerten, die jedoch alle deutlich innerhalb des zulässigen Toleranzbandes liegen. Die erfassten realen proximalen exspiratorischen Atemgasflüsse liegen einen etwa konstanten Wert niedriger als die realen proximalen inspiratorischen Atemgasflüsse, was an dem in der Beschreibungseinleitung beschriebenen Basisfluss liegt, also an einer Art Kurzschluss-Atemgasfluss von der Atemgas-Fördervorrichtung 16 unter Umgehung des Patienten 12 zum Exspirationsventil 22, dessen in die Schließrichtung vorgespannter Ventilkörper, in der Regel eine Ventilmembran, durch den Überdruck des inspiratorischen Atemgasflusses geringfügig aufgedrückt wird.

Etwa bei Atemhub Nr. 5700 tritt ein plötzlicher Fehler des proximalen Atemgas-Flusssensors 44 ein, der zu einer fehlerhaften Erfassung sowohl des inspiratorischen wie auch des exspiratorischen Atemgasflusses führt. Das erfasste reale proximale inspiratorische Atemgasvolumen steigt betragsmäßig um etwa 100 ml, das erfasste reale proximale exspiratorische Atemgasvolumens steigt betragsmäßig um etwa 110 ml an. Die realen proximalen inspiratorischen Erfassungswerte liegen damit außerhalb des Toleranzbandes, in welchem Erfassungswerte auf Grundlage eines korrekt arbeitenden proximalen Atemgas-Flusssensors erwartet würden.

Der aufgetretene Fehler kann an einer schlagartig gestiegenen Feuchtigkeitsbelastung des Strömungswiderstandsbauteils im proximalen Atemgas-Flusssensor 44 und einer dadurch bewirkten veränderten Beweglichkeit des Strömungswiderstandsbauteils im Atemgasfluss liegen.

Da der proximale Atemgas-Flusssensor 44 dann, wenn er korrekt arbeitet, dass genaueste Bild von dem dem Patienten 12 verabreichten inspiratorischen Atemgasfluss liefert, steuert die Steuervorrichtung 18 den Betrieb der Beatmungsvorrichtung 10 auf Grundlage der proximalen inspiratorischen Erfassungswerte des proximalen Atemgas-Flusssensors 44. Eine fehlerhafte Erfassung insbesondere des inspiratorischen Atemgasflusses durch den proximalen Atemgas-Flusssensor 44 hätte ohne weitere Maßnahmen eine Fehlsteuerung der Beatmungsvorrichtung 10 und damit möglicherweise gesundheitliche Schäden des Patienten 12 zur Folge.

Grundsätzlich könnte mit dem Überschreiten des Toleranzbereichs durch die proximalen inspiratorischen Erfassungswerte ein Alarm an der Ausgabevorrichtung 28 der Beatmungsvorrichtung 10 ausgegeben werden. Vorliegend führt die Steuervorrichtung 18 gemeinsam mit der Datenverarbeitungsvorrichtung 19 jedoch das in der Beschreibungseinleitung beschriebene und nachfolgend anhand des vorliegenden Ausführungsbeispiels erneut dargelegte Kalibrationsverfahren aus. Zunächst wird eine inspiratorische Kalibration durchgeführt, d. h. der proximale Atemgas-Flusssensor 44 wird hinsichtlich seiner Erfassungswerte-Ausgabecharakteristik von proximalen inspiratorischen Erfassungswerten korrigiert.

Hierzu wird zunächst eine Null-Kalibration durchgeführt, um einen Driftfehler oder Folgen eines zurückliegenden Driftfehlers am proximalen Atemgas-Flusssensor 44 zu beseitigen. Hierzu wird über nicht dargestellte Ventile in der Sensor-Leitungsanordnung 46 der Innenraum des beispielhaft als Differenzdruck-Flusssensor ausgebildeten proximalen Atemgas-Flusssensors 44 auf Umgebungsdruck gebracht, sodass über die Drucksensoren 27 kein Druckunterschied beiderseits des Strömungswiderstandsbauteils im proximalen Atemgas-Flusssensor 44 erfasst wird. Dieser Erfassungszustand stellt sich ein, wenn kein Atemgasfluss durch den proximalen Atemgas-Flusssensor 44 strömt. Der in diesem Erfassungszustand erhaltene proximale inspiratorische Erfassungswert wird auf Null gesetzt und auf Null kalibriert, sodass er in gleichen Erfassungszuständen wieder einen proximalen inspiratorischen Erfassungswert von Null ausgibt. Diese in der Fachwelt als "Autozero" bezeichnete Null-Kalibration kann sowohl regelmäßig ausgeführt werden als auch dann, wenn einer der proximalen Erfassungswerte aus inspiratorischem und exspiratorischem für eine vorbestimmte Anzahl von aufeinanderfolgenden Messungen außerhalb des Toleranzbands gelegen ist.

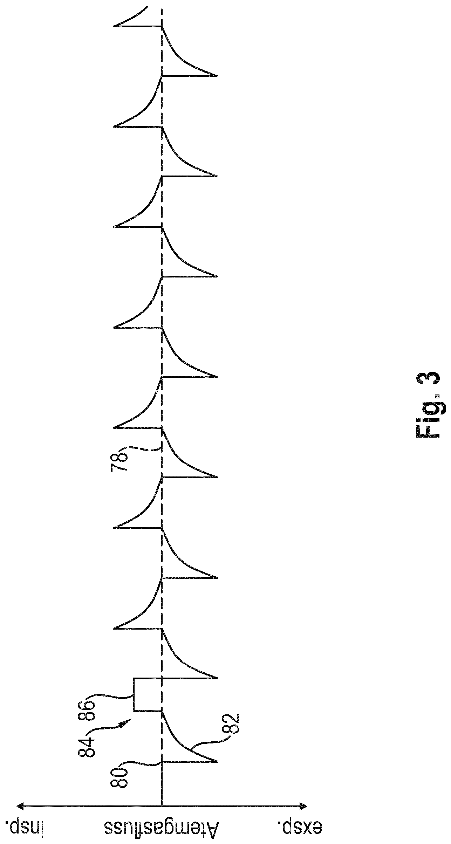

Figur 3 zeigt einen zeitlichen Verlauf des proximalen Atemgasflusses, wie er vom proximalen Atemgas-Flusssensor 44 erfasst werden könnte. Kurvenverläufe oberhalb der Nulllinie 78 in Figur 3 zeigen inspiratorische Atemgasflüsse, unterhalb der Nulllinie 78 zeigen exspiratorische Atemgasflüsse.

Nachdem das aus den realen proximalen inspiratorischen Erfassungswerten durch Integration über die Zeit ermittelte inspiratorische Atemgasvolumen wenigstens einmal, vorzugsweise mehrere Male, wie etwa genau dreimal, außerhalb des zulässigen Toleranzbereichs lag, wird der proximale Atemgas-Flusssensor 44 beispielsweise am Ende einer Inspirationsphase, etwa bei Punkt 80, durch eine Null-Kalibration korrigiert. Die Null-Kalibration kann jedoch auch an einem beliebigen anderen Betriebspunkt der Beatmungsvorrichtung 10 erfolgen.

Nach der auf die Null-Kalibration folgenden Exspirationsphase 82, die noch zum Atemhub gehört, in welchem die Null-Kalibration durchgeführt wurde (als ein Atemhub wird gemäß der vorliegenden Erfindung eine Inspirationsphase gefolgt von einer Exspirationsphase verstanden), erfolgt die inspiratorische Kalibration. In einer Kalibrations-Betriebssituation wird zunächst das Exspirationsventil 22 durch die Steuervorrichtung 18 mittels eines geeigneten Aktuators, etwa eines Stößels, geschlossen und geschlossen gehalten. Ein Basisfluss durch die Inspirationsteilschläuche 34 und 36 unmittelbar in den Exspirationsschlauch 42 ist damit reduziert oder sogar unterbunden. Der Betrieb der Konditioniervorrichtung 38 wird kurzzeitig unterbrochen.

In der ersten Inspirationsphase 84 nach dem Atemhub der Null-Kalibration wird, abweichend von den sonstigen Inspirationsphasen für die Dauer der Inspiration ein betragsmäßig konstanter inspiratorischer Atemgasfluss 86 erzeugt. Die Steuervorrichtung 18 steuert hierzu die Atemgas-Fördervorrichtung 16 entsprechend an. Zwar ist der zeitliche Verlauf des inspiratorischen Atemgasflusses anders als in Betriebsphasen der Beatmungsvorrichtung 10 außerhalb der Kalibrations-Betriebssituation, dennoch wird etwa das gleiche Tidalvolumen verabreicht.

Während der Inspirationsphase in der Kalibrations-Betriebssituation wird wiederholt der proximale inspiratorische Atemgasfluss durch den proximalen Atemgas-Flusssensor 44 erfasst und als realer proximaler inspiratorischer Erfassungswert an die Datenverarbeitungsvorrichtung 19 ausgegeben.

Gleichzeitig wird unter Verwendung eines ersten Modells, welches in Form der Gleichung 5 aus der Beschreibungseinleitung in der Datenspeicheranordnung 19a hinterlegt ist, auf Grundlage der distalen Erfassungswerte des distalen Atemgas-Flusssensors 48, welche den distalen inspiratorischen Atemgasfluss repräsentieren, ein Näherungswert $_{prox}^{approx}q(t)_{insp}$ ermittelt, welcher einen theoretischen proximalen inspiratorischen Erfassungswert angibt, den ein hypothetischer fehlerfrei arbeitender, etwa fehlerfrei hergestellter fabrikneuer, proximaler Arbeitsgas-Flusssensor unter denselben Bedingungen wie der tatsächlich arbeitende proximale Arbeitsgas-Flusssensor 44 ausgegeben würde.

Die Bestimmung von Koeffizienten in der Gleichung 5, wie etwa $^{tube}C$ kann entweder durch einen Selbsttest der Beatmungsvorrichtung 10 vor Beginn des Beatmungsbetriebs erfolgen, oder es können Anfangswerte für die Koeffizienten in der Datenspeicheranordnung 19a hinterlegt sein. Die Verwendung eines möglichst konstanten inspiratorischen Atemgasflusses während der Kalibrations-Betriebssituation vor Anschluss des Patienten an die Beatmungsvorrichtung bei gleichzeitig ausgeschalteter Konditioniervorrichtung 38 hat den Vorteil, dass die Terme der Gleichungen 2 und 3 vernachlässigbar klein werden, da bei ausgeschalteter Konditioniervorrichtung 38 kaum Feuchtigkeitsfluss $^{humid}q(t)$ und keine nennenswerte Druckänderung mit der Zeit $\dfrac{d_{prox}p(t)_{insp}}{dt}$ auftritt. Bei an die Beatmungsvorrichtung angeschlossenem Patienten wird es jedoch auch konstantem inspiratorischem Atemgasfluss einen Druckanstieg geben. Doch selbst wenn ein Feuchtigkeitsfluss und eine Druckänderung mit der Zeit mit nennenswerten Beträgen aufträten, würden diese in dem ersten Modell gemäß Gleichung 5 angemessen berücksichtigt.

Für die so erhaltenen Wertepaare aus realem proximalem inspiratorischem Atemgasfluss und theoretischem proximalem inspiratorischem Atemgasfluss wird gemäß eines in der Datenspeicheranordnung 19a hinterlegten zweiten Modells, welches beispielsweise einen linearen Zusammenhang zwischen den realen und theoretischen Werten eines Wertepaares gemäß obiger Gleichung 7 postuliert, durch lineare Regression der in der Gleichung 7 enthaltene inspiratorische Fehlerparameter $k_{insp}$ quantifiziert. Die lineare Regression kann beispielsweise die Methode der kleinsten Fehlerquadrate bei der Anpassung des inspiratorischen Fehlerparameters $k_{insp}$ nutzen.

Im Anschluss an die durchgeführte lineare Regression bewertet die Datenverarbeitungsvorrichtung 19 deren Güte, beispielsweise anhand des hierfür bekannten R-Quadrat-Werts, etwa ob der R-Quadrat-Wert eine vorbestimmte Schwelle von beispielsweise 0,75 übersteigt oder/und anhand eines Vergleichs, ob der Betrag des inspiratorischen Fehlerparameters eine vorbestimmte Schwelle, etwa von 1,3, übersteigt oder nicht. Sofern nicht wenigstens eine, vorzugsweise beide, der genannten Güteprüfungen zugunsten des quantifizierten inspiratorischen Fehlerparameters $k_{insp}$ ausfallen, wird der jüngst quantifizierte inspiratorische Fehlerparameter verworfen und entweder der zuletzt, also unmittelbar vor dem jüngst quantifizierten gültige inspiratorische Fehlerparameter weiter verwendet oder, sofern noch kein inspiratorische Fehlerparameter durch lineare Regression ermittelt wurde, dessen bevorzugter Anfangswert von 1 verwendet.

Dann, wenn die lineare Regression zur Quantifizierung des inspiratorischen Fehlerparameters $k_{insp}$ eine ausreichende Güte aufweist, wird jeder reale proximale inspiratorische Erfassungswert des proximalen Atemgas-Flusssensors 44 durch Multiplikation mit dem quantifizierten inspiratorischen Fehlerparameter $k_{insp}$ zu einem korrigierten proximalen inspiratorischen Erfassungswert geändert. Der korrigierte proximale inspiratorische Erfassungswert liegt gemäß Gleichung 6 näher bei dem theoretischen proximalen inspiratorischen Erfassungswert eines hypothetischen fehlerfrei arbeitenden proximalen Atemgas-Flusssensors als der reale proximale inspiratorische Erfassungswert. Die Genauigkeit der Bearbeitungsvorrichtung im inspiratorischen Betrieb ist dadurch erheblich verbessert. Eine Unterbrechung des Beatmungsbetriebs muss hierfür nicht erfolgen. Ebenso wenig wird für die Korrektur der realen inspiratorischen Erfassungswerte des proximalen Atemgas-Flusssensors 44 eine zusätzliche Kalibrationsvorrichtung benötigt. Die inspiratorische Kalibration des proximalen Atemgas-Flusssensors 44 kann mit den für den herkömmlichen Beatmungsbetrieb vorhandenen Bordmitteln der Beatmungsvorrichtung 10 durchgeführt werden.

Um möglichst schnell auch den in Figur 2 gezeigten Fehler in der Erfassung des proximalen exspiratorischen Atemgasflusses zu korrigieren, wird möglichst unmittelbar nach erfolgreicher Quantifizierung des inspiratorischen Fehlerparameters $k_{insp}$ mit der exspiratorischen Kalibration begonnen. Hierzu wird ein in der Datenspeicheranordnung 19a hinterlegtes drittes Modell herangezogen, welches gemäß einer bevorzugten Ausführungsform durch die obige Gleichung 8 repräsentiert ist. Strukturell entspricht die Gleichung 8 der Gleichung 7 des zweiten Modells. Dies ist technisch sinnvoll, da ein und derselbe Atemgas-Flusssensor 44, mit ein und derselben Fehlerursache belastet, nicht nur den inspiratorischen, sondern auch den exspiratorischen Atemgasfluss erfasst. Allerdings steht, anders als für die inspiratorische Kalibration, für die exspiratorische Kalibration kein zweiter Atemgas-Flusssensor zur Verfügung, dessen Werte zur Korrektur auch der Erfassung des exspiratorischen Atemgasflusses durch den proximalen Atemgas-Flusssensor 44 herangezogen werden könnten.

Daher wird zur Durchführung der exspiratorischen Kalibration des proximalen Atemgas-Flusssensors 44 dessen betragsmäßige Asymmetrie bei der Erfassung exspiratorischer und inspiratorischer Atemgasvolumina über einen einheitlichen Atemvorgang herangezogen. Der einheitliche Atemvorgang umfasst vorzugsweise mehrere Atemhübe, insbesondere mehrere unmittelbar aufeinanderfolgende Atemhübe, um die Wirkung von Artefakten abzumildern, welche vereinzelt in Atemhüben auftreten können.

Beispielsweise kann durch die Datenverarbeitungsvorrichtung 19 über drei zusammenhängende Atemhübe hinweg durch Integration einmal der proximalen inspiratorischen Atemgasflüsse, insbesondere der realen, also nicht korrigierten proximalen inspiratorischen Atemgasflüsse, und der realen proximalen exspiratorischen Atemgasflüsse ein mittleres verabreichtes reales proximales inspiratorisches Atemgasvolumen und ein mittleres abgegebenes reales proximales exspiratorisches Atemgasvolumen ermittelt werden.

Dann, wenn das mittlere verabreichte reale proximale inspiratorische Atemgasvolumen betragsmäßig größer ist als das mittlere abgegebene reale proximale exspiratorische Atemgasvolumen, kann ein Schaden, etwa eine Leckage oder auch nur der Basisfluss, an der Beatmungsvorrichtung 10 vorliegen, der für Bedienpersonal auch durch die Betriebsparameter der Beatmung erkennbar bleiben soll. Daher wird in diesem Falle der exspiratorische Fehlerparameter gleich dem bereits quantifizierten inspiratorischen Fehlerparameter gesetzt. Die vom fehlerbehafteten proximalen Atemgas-Flusssensor 44 ermittelte Asymmetrie in verabreichten und abgegebenen Atemgasvolumina, welche jedoch ihre Ursache nicht in einem Fehler des Atemgas-Flusssensors 44 hat, bleibt dadurch erhalten und für Bedienpersonal beobachtbar.

Dann, wenn das mittlere verabreichte reale proximale inspiratorische Atemgasvolumen nicht nur betragsmäßig größer ist als das mittlere abgegebene reale proximale exspiratorische Atemgasvolumen, sondern um mehr als ein vorbestimmtes Maß größer ist als das mittlere abgegebene reale proximale Atemgasvolumen, werden sowohl der inspiratorische Fehlerparameter als auch der exspiratorische Fehlerparameter auf ihre unmittelbar zuvor letzten gültigen Werte gesetzt. Dies können bei erstmaliger Quantifizierung deren Anfangswerte sein, wobei bevorzugt für sowohl den inspiratorischen Fehlerparameter als auch den exspiratorischen Fehlerparameter der Wert 1 als Anfangswert verwendet wird.

Dann, wenn das mittlere abgegebene reale proximale exspiratorische Atemgasvolumen betragsmäßig größer ist als das mittlere verabreichte reale proximale inspiratorische Atemgasvolumen, liegt dies mit an Sicherheit grenzender Wahrscheinlichkeit an einer Fehlerbelastung des proximalen Atemgas-Flusssensors 44. In diesem Fall wird der exspiratorische Fehlerparameter $k_{exsp}$ derart quantifiziert, dass der betragsmäßige Unterschied zwischen einem dem mittleren verabreichten proximalen inspiratorischen Atemgasvolumen und dem gemäß Gleichung 8 korrigierten mittleren abgegebenen theoretischen proximalen exspiratorischen Atemgasvolumen gleich Null wird oder in einem vorbestimmten Toleranzbereich um den Wert Null liegt. Das für diese Berechnung verwendete mittlere verabreichte proximale inspiratorische Atemgasvolumen kann das mittlere verabreichte reale proximale inspiratorische Atemgasvolumen sein, ist jedoch bevorzugt das mittlere verabreichte korrigierte proximale inspiratorische Atemgasvolumen, damit eine nur aufgrund eines Sensorfehlers auftretende Asymmetrie zwischen den inspiratorischen und exspiratorischen Atemgasvolumina nach Korrektur der erfassten Atemgasflüsse durch den inspiratorischen und den exspiratorischen Fehlerparameter sicher auf einen tolerierbaren Wert reduziert oder, bevorzugt, vollständig beseitigt wird.

Anschließend werden im weiteren Beatmungsbetrieb die realen proximalen exspiratorischen Erfassungswerte des proximalen Atemgas-Flusssensors 44 gemäß Gleichung 8 durch Multiplikation mit dem quantifizierten exspiratorischen Fehlerparameter $k_{exsp}$ zu mit geringerem Fehler den wahren proximalen exspiratorischen Atemgasfluss repräsentierenden korrigierten proximalen exspiratorischen Erfassungswerten umgewandelt.

Im weiteren Beatmungsbetrieb kann die Beatmungsvorrichtung 10 anhand von Plausibilitätsüberprüfungen prüfen, ob die aktuell verwendeten Werte des exspiratorischen Fehlerparameters $k_{exsp}$ und des inspiratorischen Fehlerparameters $k_{insp}$ noch gültig sind.

Zur Plausibilitätsüberprüfung des inspiratorischen Fehlerparameters $k_{insp}$ kann die Datenverarbeitungsvorrichtung 19 prüfen, ob ein über mehrere, vorzugsweise mehrere zusammenhängende, Atemhübe ermitteltes mittleres korrigiertes verabreichtes proximales inspiratorisches Atemgasvolumen betragsmäßig um mehr als einen vorbestimmten Inspirations-Toleranzwert vom mittleren realen verabreichten proximalen inspiratorischen Atemgasvolumen abweicht.

Zur Plausibilitätsüberprüfung des exspiratorischen Fehlerparameters $k_{exsp}$ kann die Datenverarbeitungsvorrichtung 19 prüfen, ob ein über mehrere, vorzugsweise mehrere zusammenhängende, Atemhübe ermitteltes mittleres korrigiertes verabreichtes proximales exspiratorisches Atemgasvolumen betragsmäßig um mehr als einen vorbestimmten ersten Exspirations-Toleranzwert vom mittleren realen verabreichten proximalen exspiratorischen Atemgasvolumen abweicht.

Zusätzlich oder alternativ kann die Datenverarbeitungsvorrichtung 19 Plausibilitätsüberprüfung des exspiratorischen Fehlerparameters $k_{exsp}$ prüfen, ob der Betrag eines Unterschieds zwischen einem über mehrere, vorzugsweise mehrere zusammenhängende, Atemhübe ermittelten mittleren realen verabreichten proximalen exspiratorischen Atemgasvolumen und einem über mehrere, vorzugsweise mehrere zusammenhängende, Atemhübe ermittelten mittleren realen verabreichten proximalen inspiratorischen Atemgasvolumen betragsmäßig um mehr als einen vorbestimmten zweiten Exspirations-Toleranzwert von einem Betrag eines Unterschieds zwischen einem über mehrere, vorzugsweise mehrere zusammenhängende, Atemhübe ermittelten mittleren korrigierten verabreichten proximalen exspiratorischen Atemgasvolumen und einem über mehrere, vorzugsweise mehrere zusammenhängende, Atemhübe ermittelten mittleren korrigierten verabreichten proximalen inspiratorischen Atemgasvolumen abweicht.

Dann, wenn die Plausibilitätsprüfung ergibt, dass wenigstens ein Fehlerparameter unplausibel ist, wird dieser auf den Wert 1 zurückgesetzt.

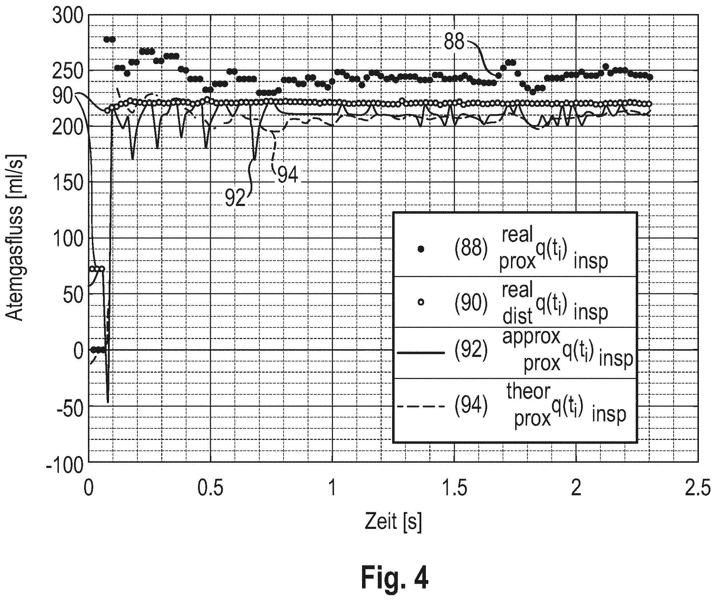

In Figur 4 ist zum besseren Verständnis der in der vorliegenden Anmeldung diskutierten Online-Kalibration des Atemgas-Flusssensors 44 während einer laufenden Beatmung eine Grafik dargestellt, welche über 2,5 Sekunden hinweg einen inspiratorischen Beatmungsbetrieb und die währenddessen erfassten bzw. ermittelten Beatmungsdaten zeigt, welche die Datenverarbeitungsvorrichtung 19 von den entsprechenden Sensoren übertragen erhält bzw. durch Rechenvorgänge ermittelt.

In Figur 4 zeigt der Punkteverlauf 88 die tatsächlich vom proximalen Atemgas-Flusssensor 44 erfassten Atemgas-Flusswerte. Dies entspricht den Werten $_{prox}^{real}q(t)_{insp}$ in Gleichung 6 bzw. $_{prox}^{real}q(t_i)_{insp}$ in Gleichung 7.

In Figur 4 zeigt der Punkteverlauf 90 die tatsächlich vom distalen Atemgas-Flusssensor 48 erfassten Atemgas-Flusswerte. Dies entspricht den Werten $_{dist}q(t)_{insp}$ in den Gleichungen 1 und 5. Zur eindeutigen Zuordnung wurde diesem Wert als tatsächlicher Messwert den Index "real" beigestellt. Die Bezeichnung der distalen Messwerte in Fig. 4 entspricht somit strukturell der Bezeichnung der proximalen Messwerte.

Die durchgezogene Linie 92 in Figur 4 zeigt die gemäß dem ersten Modell, beispielsweise repräsentiert durch die Gleichungen 4 oder 5, näherungsweise berechneten Werte für den inspiratorischen proximalen Atemgasfluss. Sie entsprechen beispielsweise den Werten $_{prox}^{approx}q(t)_{insp}$ in Gleichung 4 oder 5 oder als diskrete Werte zu diskreten

Zeitpunkten den Werten $\underset{prox}{approx}q(t_i)_{insp}$ in Gleichung 7.

Die gestrichelte Linie 94 in Figur 4 zeigt schließlich jene Atemgasflusswerte an, die ein hypothetischer, fehlerfrei arbeitender gleichartiger proximaler Atemgas-Flusssensor wie der aktuell verwendete proximale Atemgas-Flusssensor 44 in derselben Betriebssituation liefern würde. Die Werte der Linie 94 entsprechen beispielsweise den obigen Werten $\underset{prox}{theor}q(t)_{insp}$ gemäß Gleichung 6. Diese Werte sind mit dem inspiratorischen Fehlerparameter $k_{insp}$ korrigierte reale Messwerte $\underset{prox}{real}q(t)_{insp}$ des proximalen Atemgas-Flusssensors 44.

Wie in Figur 4 erkennbar ist, sind ab dem Zeitpunkt 0,1 s die vom proximalen Atemgas-Flusssensor 44 erfassten proximalen Atemgas-Flusswerte 88 betragsmäßig größer als die gleichzeitig vom distalen Atemgas-Flusssensor 48 gemessenen distalen Atemgas-Flusswerte 90. Betragsmäßig höhere erfasste proximale Atemgas-Flusswerte 88 als erfasste distale Atemgas-Flusswerte 90, jeweils gemessen als Volumen pro Zeit, können aufgrund von Erwärmung des Atemgases auf seinem Weg zum Patienten oder/und aufgrund von Befeuchtung des Atemgases auftreten.

Da jedoch die Erwärmung und die Befeuchtung des Atemgases auf dem Weg vom distalen Flusssensor zum Patienten 12 im ersten Modell berücksichtigt sind, siehe etwa Gl. 5, sollten die vom proximalen Atemgas-Flusssensor 44 erfassten proximalen Atemgas-Flusswerte 88 betragsmäßig etwa bei den gemäß dem ersten Modell erhaltenen Werten des Graphen 92 liegen. Die Tatsache, dass in Figur 4 die erfassten proximalen Atemgas-Flusswerte 88 etwa 15 % über den gemäß dem ersten Modell ermittelten Näherungswerten 92 liegen, kann auf eine fehlerhafte Erfassung des proximalen Atemgas-Flusses hindeuten.

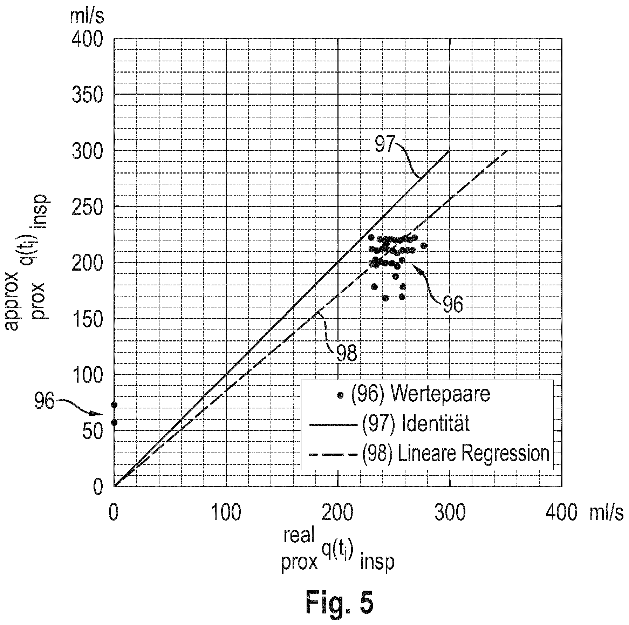

Figur 5 zeigt beispielhaft, wie ausgehend von den in Figur 4 gezeigten gemessenen proximalen Atemgas-Flusswerten, also etwa $\underset{prox}{real}q(t_i)_{insp}$ , und den gemäß dem ersten Modell berechneten Näherungswerten, also etwa $\underset{prox}{approx}q(t_i)_{insp}$ , gemäß Gleichung 7 durch lineare Regression der inspiratorische Fehlerparameter $k_{insp}$ ermittelt wird.

Das zweite Modell postuliert einen linearen Zusammenhang zwischen einem fehlerhaft gemessenen Wert $\underset{prox}{real}q(t_i)_{insp}$ und dem zum selben Messzeitpunkt theoretisch korrekten Wert $\underset{prox}{theor}q(t)_{insp}$ .

Unter der Annahme, dass die gemäß dem ersten Modell berechneten Näherungswerte betragsmäßig sehr nahe bei den theoretischen Messwerten des hypothetischen, fehlerfrei arbeitenden gleichartigen proximalen Atemgas-Flusssensors liegen, können die Wertepaare aus $\underset{prox}{real}q(t_i)_{insp}$ und $\underset{prox}{approx}q(t_i)_{insp}$ für jeweils zu gleichen Zeitpunkten $t_i$ ermittelte Werte gemäß dem zweiten Modell, etwa gemäß Gleichung 7, zur Bestimmung des inspiratorischen Fehlerparameters $k_{insp}$ herangezogen werden.

Würde der aktuell eingesetzte proximale Atemgas-Flusssensor 44 korrekt arbeiten, dann würde die Punktewolke aus Messwertepaaren 96 etwa auf der Winkelhalbierenden 97 zwischen Abszisse und Ordinate von Figur 5, liegen, längs welcher gemessene und näherungsweise berechnete Werte identisch sind. Bei dem in Fig. 4 gezeigten etwa konstanten Volumenstrom bilden die Messwertepaare eine Punktewolke 96. Bei sich mit der Zeit betragsmäßig verändernden Atemgasströmen würde die Punktewolke mit zunehmender betragsmäßiger Veränderung bei fehlerfreier Erfassung durch den proximalen Atemgas-Flusssensor 44 zunehmend längs der Winkelhalbierenden 97 verlaufen. Die Lage der Punktewolke 96 weit überwiegend auf einer Seite der Winkelhalbierenden 97 zeigt an, dass der aktuell eingesetzte proximale Atemgas-Flusssensor 44 aller Wahrscheinlichkeit nach nicht korrekt arbeitet.

Durch lineare Regression wird gemäß dem zweiten Modell die strichlinierte Gerade 98 als Näherungsgerade für die Messwertepaare 96 ermittelt. Deren Steigung ist im Gegensatz zur Identitätssteigung "1" der Winkelhalbierenden 97 der Betrag des inspiratorischen Fehlerparameters $k_{insp}$, welcher nach dessen Ermittlung gemäß Gleichung 6 zur Fehlerkorrektur der gemessenen Atemgas-Flusswerte $\underset{prox}{real}q(t_i)_{insp}$ herangezogen wird.

Mit üblichen mathematischen Methoden kann die Güte der durchgeführten Regression beurteilt werden. Damit einher geht eine Beurteilung, wie gut die Näherungsgerade 98 die Messwertepaare 96 annähert. Anhand von in der Beschreibungseinleitung beschriebenen Güteschwellenwerten kann die Datenverarbeitungsvorrichtung 19 entscheiden, ob die Online-Kalibration durch den inspiratorischen Fehlerparameter $k_{insp}$ angewendet oder verworfen werden soll.

**Patentansprüche**

1. Beatmungsvorrichtung (10) zur wenigstens unterstützenden künstlichen Beatmung eines Patienten (12), umfassend:

   - eine Atemgasleitung (30) zur Leitung von inspiratorischem Atemgas zu dem Patienten (12) hin und zur Leitung von exspiratorischem Atemgas vom Patienten (12) weg,
   - eine Atemgas-Fördervorrichtung (16) zur Förderung von inspiratorischem Atemgas in der Atemgasleitung (30),
   - einen näher am patientenseitigen Ende der Atemgasleitung (30) angeordneten proximalen Atemgas-Flusssensor (44) zur quantitativen Erfassung eines inspiratorischen und eines exspiratorischen proximalen Atemgasflusses in der Atemgasleitung (30) und zur Ausgabe entsprechender Erfassungswerte,
   - einen weiter vom patientenseitigen Ende der Atemgasleitung (30) entfernt angeordneten distalen Atemgas-Flusssensor (48) zur quantitativen Erfassung eines distalen Atemgasflusses in der Atemgasleitung (30),
   - eine Datenverarbeitungsvorrichtung (19), welche zur Verarbeitung von an die Datenverarbeitungsvorrichtung (19) ausgegebenen Erfassungswerten des proximalen (44) und des distalen Atemgas-Flusssensors (48) ausgebildet ist,
   - eine mit der Datenverarbeitungsvorrichtung (19) in Datenübertragungsverbindung stehende Datenspeicheranordnung (19a), wobei in der Datenspeicheranordnung (19a) ein erstes Modell hinterlegt ist, welches als ein Betriebsmodell gestattet, für ein und dieselbe Betriebssituation der Beatmungsvorrichtung (10) einen Atemgasfluss aus proximalem Atemgasfluss im Bereich des proximalen Atemgas-Flusssensors (44) und distalem Atemgasfluss im Bereich des distalen Atemgas-Flusssensors (48) auf Grundlage des jeweils anderen Atemgasflusses zu ermitteln,
   - eine Steuervorrichtung (18) zur Steuerung wenigstens der Atemgas-Fördervorrichtung (16) und der Datenverarbeitungsvorrichtung (19),
   wobei die Datenverarbeitungsvorrichtung (19) dazu ausgebildet ist, Näherungswerte für Erfassungswerte des proximalen Atemgas-Flusssensors (44) in einer Betriebssituation der Beatmungsvorrichtung (10) nach Maßgabe des ersten Modells auf Grundlage von in der Betriebssituation erfassten Erfassungswerten des distalen Atemgas-Flusssensors (48) näherungsweise zu ermitteln,
   **dadurch gekennzeichnet, dass** der distale Atemgas-Flusssensor (48) zur quantitativen Erfassung des distalen inspiratorischen Atemgasflusses ausgebildet ist und dass in der Datenspeicheranordnung (19) ein zweites Modell hinterlegt ist, welches als ein inspiratorisches Fehlermodell gestattet, für ein und dieselbe Betriebssituation einen Erfassungswert aus einem theoretischen inspiratorischen Erfassungswert, wie er von einem hypothetischen, fehlerfrei arbeitenden proximalen Atemgas-Flusssensor als Ergebnis einer Erfassung des inspiratorischen proximalen Atemgasflusses an die Datenverarbeitungsvorrichtung (19) ausgegeben würde, und einem realen inspiratorischen Erfassungswert, wie er vom proximalen Atemgas-Flusssensor (44) als Ergebnis einer Erfassung des proximalen inspiratorischen Atemgasflusses tatsächlich an die Datenverarbeitungsvorrichtung (19) ausgegeben wird, unter Verwendung wenigstens eines inspiratorischen Fehlerparameters auf Grundlage des jeweils anderen Erfassungswerts zu ermitteln,
   wobei die Datenverarbeitungsvorrichtung (19) dazu ausgebildet ist, auf Grundlage eines vom distalen Atemgas-Flusssensor (48) an die Datenverarbeitungsvorrichtung (19) ausgegeben Erfassungswerts, welcher einen inspiratorischen distalen Atemgasfluss in einer Kalibrations-Betriebssituation repräsentiert, nach Maßgabe des ersten Modells einen inspiratorischen Kalibrations-Näherungswert zu ermitteln, welcher einen vom proximalen Atemgas-Flusssensor (44) bei einer Erfassung des proximalen inspiratorischen Atemgasflusses in der Kalibrations-Betriebssituation erwarteten inspiratorischen Erfassungswert repräsentiert,
   wobei die Datenverarbeitungsvorrichtung (19) weiter dazu ausgebildet ist, unter Verwendung des Kalibrations-Näherungswerts als dem theoretischen inspiratorischen Erfassungswert und eines in der Kalibrations-Betriebssituation vom proximalen Atemgas-Flusssensor (44) an die Datenverarbeitungsvorrichtung (19) ausgegebenen realen inspiratorischen Erfassungswerts nach Maßgabe des zweiten Modells den wenigstens einen inspiratorischen Fehlerparameter zu quantifizieren, und
   wobei die Datenverarbeitungsvorrichtung (19) noch weiter dazu ausgebildet ist, im weiteren Betrieb der Beatmungsvorrichtung (10) den wenigstens einen quantifizierten inspiratorischen Fehlerparameter auf vom proximalen Atemgas-Flusssensor (44) an die Datenverarbeitungsvorrichtung (19) ausgegebene reale inspiratorische Erfassungswerte anzuwenden, um die tatsächlichen inspiratorischen Erfassungswerte des proximalen Atemgas-Flusssensors (44) in betragsmäßig weniger stark fehlerbehaftete korrigierte inspiratorische Erfassungswerte umzuwandeln.

2. Beatmungsvorrichtung (10) nach Anspruch 1,
   **dadurch gekennzeichnet, dass** die Kalibrations-Betriebssituation eine Betriebssituation in einem gewöhnlichen Beatmungsbetrieb ist, während welchem eine Kalibration des proximalen Atemgas-Flusssensors (44) durch Quantifizierung des wenigstens einen inspiratorischen Fehlerparameters zur Korrektur der vom proximalen Atemgas-Flusssensor (44) erfassten inspiratorischen Flusswerte durchgeführt wird.

3. Beatmungsvorrichtung (10) nach Anspruch 1 oder 2,
**dadurch gekennzeichnet, dass** wenigstens eine Vorrichtung aus Steuervorrichtung (18) und Datenverarbeitungsvorrichtung (19) dazu ausgebildet ist, eine Null-Kalibration der Erfassungswerte-Ausgabecharakteristik des proximalen Atemgas-Flusssensors (44) zur Vermeidung oder Korrektur eines Driftfehlers derart durchzuführen, dass der proximale Atemgas-Flusssensor (44) unmittelbar nach Durchführung der Null-Kalibration unter Erfassungsbedingungen, die einem Fehlen eines Gasflusses durch den proximalen Atemgas-Flusssensor (44) entsprechen, einen innerhalb eines vorgegebenen Toleranzintervalls um einen Flusswert von Null gelegenen Erfassungswert ausgibt.

4. Beatmungsvorrichtung (10) nach Anspruch 3,
**dadurch gekennzeichnet, dass** wenigstens eine Vorrichtung aus Steuervorrichtung (18) und Datenverarbeitungsvorrichtung (19) dazu ausgebildet ist, eine Quantifizierung des wenigstens einen inspiratorischen Fehlerparameters mit oder in einem von drei Atemhüben zu beginnen, welche auf eine Durchführung einer Null-Kalibration folgen.

5. Beatmungsvorrichtung (10) nach einem der vorhergehenden Ansprüche,
**dadurch gekennzeichnet, dass** die Beatmungsvorrichtung (10) im Exspirationspfad (42) der Atemgasleitung (30) ein Exspirationsventil (22) aufweist, wobei die Steuervorrichtung (18) dazu ausgebildet ist, in der Kalibrations-Betriebssituation für die Erfassung des einen distalen inspiratorischen Atemgasfluss repräsentierenden Erfassungswerts des distalen Atemgas-Flusssensors (48) und für die Erfassung des realen inspiratorischen Erfassungswerts des proximalen Atemgas-Flusssensors (44) zu schließen oder/und geschlossen zu halten.

6. Beatmungsvorrichtung (10) nach einem der vorhergehenden Ansprüche,
**dadurch gekennzeichnet, dass** die Beatmungsvorrichtung (10) im Inspirationspfad (32) der Atemgasleitung (30) eine Konditioniervorrichtung (38) zur Änderung der Temperatur oder/und der absoluten Feuchtigkeit des inspiratorischen Atemgases oder/und zur Beimischung von Aerosolen in das inspiratorische Atemgas aufweist, wobei die Steuervorrichtung (18) dazu ausgebildet ist, in der Kalibrations-Betriebssituation den Betrieb der Konditioniervorrichtung (38) zu beenden oder zu unterbrechen.

7. Beatmungsvorrichtung (10) nach einem der vorhergehenden Ansprüche,
**dadurch gekennzeichnet, dass** das erste Modell eine quantitative Änderung des Atemgasflusses auf Grundlage einer Änderung der Temperatur oder/und des Drucks oder/und der Feuchtigkeit des Atemgases zwischen dem Anordnungsbereich des distalen Atemgas-Flusssensors (48) und dem Anordnungsbereich des proximalen Atemgas-Flusssensors (44) oder/und auf Grundlage einer Elastizität der Atemgasleitung (30) berücksichtigt.

8. Beatmungsvorrichtung (10) nach einem der vorhergehenden Ansprüche,
**dadurch gekennzeichnet, dass** das erste Modell ein mathematisch-funktioneller Zusammenhang zwischen dem proximalen Atemgasfluss im Anordnungsbereich des proximalen Atemgas-Flusssensors (44) und dem distalen Atemgasfluss im Anordnungsbereich des distalen Atemgas-Flusssensors (48) ist.

9. Beatmungsvorrichtung (10) nach einem der vorhergehenden Ansprüche,
**dadurch gekennzeichnet, dass** das zweite Modell ein mathematisch-funktioneller, insbesondere linearer, Zusammenhang zwischen einem realen inspiratorischen Erfassungswert des tatsächlich in der Atemgasleitung (30) angeordneten proximalen Atemgas-Flusssensors (44) und einem theoretischen inspiratorischen Erfassungswert bei hypothetischer Erfassung derselben Strömungssituation durch den hypothetischen, fehlerfrei arbeitenden proximalen Atemgas-Flusssensors ist, wobei der wenigstens eine Koeffizient des mathematisch-funktionellen Zusammenhangs der wenigstens eine inspiratorische Fehlerparameter ist.

10. Beatmungsvorrichtung (10) nach Anspruch 9,
**dadurch gekennzeichnet, dass** die Datenverarbeitungsvorrichtung (19) dazu ausgebildet ist, den wenigstens einen inspiratorischen Fehlerparameter durch ein Regressionsverfahren zu quantifizieren.

11. Beatmungsvorrichtung (10) nach Anspruch 10,
**dadurch gekennzeichnet, dass** die Datenverarbeitungsvorrichtung (19) dazu ausgebildet ist, einen die Anpassungsgüte der zur Quantifizierung des wenigstens einen Fehlerparameters durchgeführten Regression repräsentierenden Gütewert zu ermitteln und mit einem vorbestimmten, eine Mindest-Güte repräsentierenden Güte-Schwellenwert zu vergleichen, wobei die Datenverarbeitungsvorrichtung (19) bevorzugt dazu ausgebildet ist, die Quantifizierung des wenigstens einen inspiratorischen Fehlerparameters zu verwerfen, wenn der Gütewert den Güte-Schwellenwert nicht wenigstens erreicht.

12. Beatmungsvorrichtung (10) nach einem der vorhergehenden Ansprüche, **dadurch gekennzeichnet, dass** die Datenverarbeitungsvorrichtung (19) dazu ausgebildet ist, die Quantifizierung des wenigstens einen inspiratorischen Fehlerparameters zu verwerfen, wenn der wenigstens eine inspiratorische Fehlerparameter nicht wenigstens einen vorbestimmten Mindest-Fehlerparameterwert erreicht.

13. Beatmungsvorrichtung (10) nach einem der vorhergehenden Ansprüche,

**dadurch gekennzeichnet, dass** in der Datenspeicheranordnung (19a) ein drittes Modell hinterlegt ist, welches als ein exspiratorisches Fehlermodell gestattet, für ein und dieselbe Betriebssituation einen Erfassungswert aus einem theoretischen exspiratorischen Erfassungswert, wie er von einem hypothetischen, fehlerfrei arbeitenden proximalen Atemgas-Flusssensor als Ergebnis einer Erfassung des exspiratorischen proximalen Atemgasflusses an die Datenverarbeitungsvorrichtung (19) ausgegeben würde, und einem realen exspiratorischen Erfassungswert, wie er vom proximalen Atemgas-Flusssensor (44) als Ergebnis einer Erfassung des proximalen exspiratorischen Atemgasflusses tatsächlich an die Datenverarbeitungsvorrichtung (19) ausgegeben wird, unter Verwendung wenigstens eines exspiratorischen Fehlerparameters auf Grundlage des jeweils anderen Erfassungswerts zu ermitteln, wobei die Datenverarbeitungsvorrichtung (19) dazu ausgebildet ist, auf Grundlage von den inspiratorischen Atemgasfluss repräsentierenden inspiratorischen Erfassungswerten des proximalen Atemgas-Flusssensors (44) sowie auf Grundlage von den exspiratorischen Atemgasfluss repräsentierenden realen exspiratorischen Erfassungswerten des proximalen Atemgas-Flusssensors (44) für wenigstens einen Atemhub ein dem Patienten (12) verabreichtes inspiratorisches Atemgasvolumen und ein vom Patienten (12) abgegebenes reales exspiratorisches Atemgasvolumen zu ermitteln,
wobei die Datenverarbeitungsvorrichtung (19) weiter dazu ausgebildet ist, nach Maßgabe des dritten Modells den wenigstens einen exspiratorischen Fehlerparameter derart zu quantifizieren, dass ein betragsmäßiger Unterschied zwischen dem verabreichten inspiratorischen Atemgasvolumen und einem nach Maßgabe des dritten Modells ermittelten abgegebenen theoretischen exspiratorischen Atemgasvolumen innerhalb eines vorbestimmten Toleranzbereichs um den Wert Null gelegen ist,
wobei die Datenverarbeitungsvorrichtung (19) noch weiter dazu ausgebildet ist, im weiteren Betrieb der Beatmungsvorrichtung (10) den wenigstens einen quantifizierten exspiratorischen Fehlerparameter auf vom proximalen Atemgas-Flusssensor (44) an die Datenverarbeitungsvorrichtung (19) ausgegebene reale exspiratorische Erfassungswerte anzuwenden, um die tatsächlichen exspiratorischen Erfassungswerte des proximalen Atemgas-Flusssensors (44) in betragsmäßig weniger stark fehlerbehaftete korrigierte exspiratorische Erfassungswerte umzuwandeln.

14. Beatmungsvorrichtung (10) nach Anspruch 13,
**dadurch gekennzeichnet, dass** die Datenverarbeitungsvorrichtung (19) dazu ausgebildet ist, das ermittelte verabreichte inspiratorische Atemgasvolumen auf Grundlage

a) von Näherungswerten für Erfassungswerte des proximalen Atemgas-Flusssensors (44) nach Maßgabe des ersten Modells auf Grundlage von in der Betriebssituation erfassten Erfassungswerten des distalen Atemgas-Flusssensors (48),
oder
b) von korrigierten inspiratorischen Erfassungswerten des proximalen Atemgas-Flusssensors (44)
oder
c) von realen inspiratorischen Erfassungswerten des proximalen Atemgas-Flusssensors (44)

zu ermitteln.

15. Beatmungsvorrichtung (10) nach Anspruch 13 oder 14,
**dadurch gekennzeichnet, dass** die Datenverarbeitungsvorrichtung (19) dazu ausgebildet ist, nach der Ermittlung des verabreichten inspiratorischen Atemgasvolumens und des vom Patienten (12) abgegebenen realen exspiratorischen Atemgasvolumens die beiden Atemgasvolumina betragsmäßig zu vergleichen und abhängig vom Vergleichsergebnis wenigstens eine der folgenden Maßnahmen auszuführen:

i) dann, wenn das verabreichte inspiratorische Atemgasvolumen betragsmäßig größer ist als das abgegebene reale exspiratorische Atemgasvolumen, den wenigstens einen exspiratorischen Fehlerparameter zu quantifizieren, indem er betragsmäßig gleich dem wenigstens einen inspiratorischen Fehlerparameter gesetzt wird,

ii) dann, wenn das verabreichte inspiratorische Atemgasvolumen um ein vorbestimmtes Maß betragsmäßig größer ist als das abgegebene exspiratorische Atemgasvolumen, sowohl den wenigstens einen exspiratorischen Fehlerparameter als auch den wenigstens einen inspiratorischen Fehlerparameter auf den für den jeweiligen Parameter zuletzt gültigen Wert zu setzen,

iii) dann, wenn das verabreichte inspiratorische Atemgasvolumen betragsmäßig kleiner ist als das abgegebene reale exspiratorische Atemgasvolumen, nach Maßgabe des dritten Modells den wenigstens einen exspiratorischen Fehlerparameter derart zu quantifizieren, dass ein betragsmäßiger Unterschied zwischen dem verabreichten inspiratorischen Atemgasvolumen und einem nach Maßgabe des dritten Modells ermittelten abgegebenen theoretischen exspiratorischen Atemgasvolumen innerhalb eines vorbestimmten Toleranzbereichs um den Wert Null gelegen ist.

Fig. 1

Fig. 2

Fig. 3

Fig. 4

Fig. 5

# EP 4 109 466 A1

**Europäisches Patentamt**
**European Patent Office**
**Office européen des brevets**

# EUROPÄISCHER RECHERCHENBERICHT

**Nummer der Anmeldung**

**EP 22 16 4605**

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2019/255271 A1 (BORRELLO MICHAEL ANTHONY [US]) 22. August 2019 (2019-08-22) | 1-4,6-15 | INV. G16H40/60 |
| Y | * Absätze [0001], [08ff], [0049], [37ff], [0059], [0011], [0017] * | 6 | |
| A | US 2011/146681 A1 (JAFARI MEHDI M [US] ET AL) 23. Juni 2011 (2011-06-23) * Absätze [0002], [0005], [0017], [0024], [0121]; Abbildung 1 * | 1-15 | |
| Y | US 2016/256660 A1 (AUSTIN BENJAMIN MATTHEW [AU] ET AL) 8. September 2016 (2016-09-08) * Absätze [0047], [0054], [0063], [0249] * | 6 | |
| A | CA 2 914 406 A1 (KLEIN MICHAEL [CA]; FISHER JOSEPH [CA]) 11. Dezember 2014 (2014-12-11) * Absätze [0004], [0009], [0018], [0046], [0048], [0095] * | 1-15 | |
| A | US 2021/052839 A1 (LI KUN [US] ET AL) 25. Februar 2021 (2021-02-25) * das ganze Dokument * | 1-15 | RECHERCHIERTE SACHGEBIETE (IPC) G16H |
| A | CN 112 672 778 A (HAMILTON MEDICAL AG) 16. April 2021 (2021-04-16) * das ganze Dokument * | 1-15 | |

Der vorliegende Recherchenbericht wurde für alle Patentansprüche erstellt

| Recherchenort | Abschlußdatum der Recherche | Prüfer |
|---|---|---|
| München | 6. September 2022 | Laub, Christoph |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

                      

& : Mitglied der gleichen Patentfamilie, übereinstimmendes Dokument

EPO FORM 1503 03.82 (P04C03)

31

## ANHANG ZUM EUROPÄISCHEN RECHERCHENBERICHT
## ÜBER DIE EUROPÄISCHE PATENTANMELDUNG NR.

EP 22 16 4605

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

06-09-2022

| Im Recherchenbericht angeführtes Patentdokument | Datum der Veröffentlichung | Mitglied(er) der Patentfamilie | | Datum der Veröffentlichung |
|---|---|---|---|---|
| US 2019255271 A1 | 22-08-2019 | CN | 109996579 A | 09-07-2019 |
| | | EP | 3515540 A1 | 31-07-2019 |
| | | JP | 7120996 B2 | 17-08-2022 |
| | | JP | 2019528901 A | 17-10-2019 |
| | | US | 2019255271 A1 | 22-08-2019 |
| | | WO | 2018050676 A1 | 22-03-2018 |
| US 2011146681 A1 | 23-06-2011 | US | 2011146681 A1 | 23-06-2011 |
| | | WO | 2011078944 A1 | 30-06-2011 |
| US 2016256660 A1 | 08-09-2016 | EP | 3062859 A1 | 07-09-2016 |
| | | NZ | 718540 A | 25-06-2021 |
| | | NZ | 757947 A | 25-06-2021 |
| | | US | 2016256660 A1 | 08-09-2016 |
| | | WO | 2015061848 A1 | 07-05-2015 |
| CA 2914406 A1 | 11-12-2014 | CA | 2914406 A1 | 11-12-2014 |
| | | EP | 3003446 A1 | 13-04-2016 |
| | | WO | 2014194401 A1 | 11-12-2014 |
| US 2021052839 A1 | 25-02-2021 | EP | 4018456 A1 | 29-06-2022 |
| | | US | 2021052839 A1 | 25-02-2021 |
| | | WO | 2021041137 A1 | 04-03-2021 |
| CN 112672778 A | 16-04-2021 | CN | 112672778 A | 16-04-2021 |
| | | DE | 102018214557 A1 | 05-03-2020 |
| | | EP | 3843821 A1 | 07-07-2021 |
| | | JP | 2021534898 A | 16-12-2021 |
| | | US | 2021322711 A1 | 21-10-2021 |
| | | WO | 2020043671 A1 | 05-03-2020 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 20110146681 A1
- EP 3384948 A1